# EUROPEAN PATENT APPLICATION

(11) **EP 1 297 852 A1**
(43) Date of publication of application: **02.04.2003**
(21) Application number: 01943839.9
(22) Date of filing: 27.06.2001
(51) Int. Cl.: A61K 48/00, A61K 35/76, C12N 15/86, C12N 7/00

(54) **VIRUS VECTOR FOR INTRODUCING GENE INTO RENAL CELLS**

(30) Priority: 27.06.2000 JP 2000197870
(71) Applicant: Dnavec Research Inc., Ibaraki 305-0856 (JP)
(72) Inventor: IMAI, Enyu, Takarazuka-shi, Hyogo 665-0005 (JP); ISAKA, Yoshitaka, Ikeda-shi, Osaka 563-0047 (JP); FUKUMURA, Masayuki, Ikeda-shi, Osaka 563-0022 (JP); HASEGAWA, Mamoru, c/o DNAVEC Research Inc., Tukuba-shi, Ibaraki 305-0856 (JP)
(74) Representative: VOSSIUS & PARTNER
(86) International application number: JP0105513
(87) International publication number: WO02000264

(57) **Abstract**

The present invention provides a viral vector that ensures highly efficient gene transfer into renal cells and its use. The use of a Paramyxovirus vector enables gene transfer into renal cells with high efficiency. The gene transferred by *in vivo* administration can be continuously expressed in the renal cells over a prolonged period. The vector of the present invention is suitably used in gene therapy for kidney.

## Description

### Technical Field

The present invention relates to Paramyxovirus vectors to introduce genes into renal cells.

### Background Art

In recent years, the technology of gene transfer into mammalian cells has been developed, and there has been a growing interest in treating various human diseases through gene therapy. Gene transfer into the living body is performed *in vivo* or *ex vivo*. The *in-vivo* gene transfer comprises administering a vector containing an insert such as a gene into the living body, while the *ex-vivo* transfer comprises introducing a gene into cells derived from a patient or other cells and then returning them in the body.

Unlike gene transfer into cells such as cultured cells, in general, it is difficult to transfer genes into living organs. In particular, mammalian kidney comprises structurally complicated tissues with arteries, veins, renal tubules, glomeruli, and others, and its metabolic turnover is relatively lower. Thus, it has been difficult to transfer genes into this organ with sufficient efficiency. The structure of kidney is important for maintaining its normal function, and thus gene transfer should be performed without damaging the organ or tissues. For gene transfer into kidney cells, it should be noted that it is difficult to introduce genes with high efficiency into cells derived from a glomerulus, a renal tubule and an interstitium as targets.

Introduction of a gene into a kidney has been performed by, for example, a method using HVJ (hemagglutinating virus of Japan; Sendai virus) and liposome (Tomita, N. et al., Biochem. Biophys. Res. Commun. 186: 129-134, 1992). Liposome containingcomprising HMG-1 (high mobility group 1) protein and an expression plasmid vector for SV40 large T antigen gene was prepared, and then these were injected into rat kidney from the renal artery via a catheter to introcduce the gene into the kidney. The result showed that the expression of SV40 large T antigen was succesfully detected in 15% of glomerular capillary vascular cells by the immunohistochemical method on the fourth day after gene transter. However, the expression level reached a peak in serveral days after the introduction and then dropped, and only the glomerular cells expressed the gene. Similar experiments using HVJ-liposome have been conducted to introduce the human CD59 gene into dog kidney (Akami, T. et al., Transplantation Proceedings 26: 1315-1317, 1994). However, the expression of CD59 gene in the glomerular cells was only transient and its level was not sufficiently high. For HVJ-liposome-based gene transfer, efforts have been made to improve the method of preparing liposome (Tsujie, M. et al., Kidney Int. 57: 1973-1980, 2000). However, the gene transfer efficiency is still unsatisfactory. In addition, the method of preparing HVJ-liposome is complicated, and prepared compositions have no sufficient stability.

Zhu et al. reported gene transfer into various tissues of adult mouse using a mixture of cationic liposome and plasmid DNA (Zhu, N. et al., Science 261(5118): 209-11, 1993). Transient expression was detected in cells derived from tissues such as lung, spleen, lymph nodes, bone marrow, and others after injection of the vector mixture into the blood vessels. Furthermore, genes were transferred into 25% to 50% of kidney endothelial cells. The report, however, mention neither possibility of gene transfer into glomerular cells, nor the duration of expression.

An adenoviral vector was reportedly used as a viral vector to achieve gene transfer into kidney (Moullier, P. et al., Kidney Int. 45(4): 1220-5, 1994). The β-galactosidase gene was introduced into adult rat kidney by perfusion from the renal artery, a replication-deficient adenovirus containing the β-galactosidase gene, or injection of the virus to renal pelvis *via* a catheter to cause backflow. In the case of the perfusion from the renal artery, low-level transient expression of β-galactosidase was observed. In contrast, in the case of the injection for backflow, the expression was observed in the renal papilla and medullary renal tubule, but not in kidney endothelial cells. In general, infection *via* intravascular injection of adenoviral vectors does not ensure their sufficient infectivity to renal cells. Some modifications have been made to improve the methods of adenoviral gene transfer (U.S. patent No. 5,869,230). This patent describes that a kidney is cooled on ice or such to prevent ischemic injury during vector transfer; that oxygen shortage in cells is prevented by using an oxygen-supplying agent such as hemoglobin; that vector infection can be regulated by using a vasodilator such as dopamine; and that a vector can be introduced into 5% to 15% of kidney endothelial cells by this method.

However, adenoviral vectors have been reported to induce relatively high immune reactions. After initial non-specific inflammation reaction, the transfected cells were attacked through cytotoxic T lymphocyte-mediated specific immune reaction. Efforts have been made to develop adenoviral vectors with reduced immunogenicities through gene deletions and modifications, but are not still successful.

Viral vectors for gene therapy include retroviral vectors. Retroviral vectors have an advantage of ensuring long-term stable expression of a gene introduced by integrating the gene into host chromosome, but in general, their target is restricted to only certain cells and should be dividing cells. Since most of kidney cells are non-dividing cells that have been terminally differentiated, retroviral vector-mediated gene therapy is limited mainly in *ex-vivo* approaches. Pseudotyped vectors and lentivirus-based vectors have been developed, but such vectors have risks of activation of oncogenes and destruction of tumor suppression genes due to random chromosomal insertion. Other defects include difficulties of preparation of high titer viral vectors.

### Disclosure of the Invention

An objective of the present invention is to provide a viral vector that ensures high efficiency gene transfer into renal cells and the use thereof. More specifically, the present invention provides a Paramyxovirus vector for introducing genes into renal cells, a composition comprising the vector for gene transfer into renal cells, and a method of introducing genes into renal cells using the vector.

In recent years, recombinant viral vectors for gene transfer based on Paramyxoviruses including Sendai virus have been developed by using technologies of reverse genetics for the viruses (Kato, A. et al., Genes Cells 1: 569-579, 1996; Kato, A. et al., EMBO J. 16: 578-587, 1997; Yu, D. et al., Genes Cells 2: 457-466, 1997; Hasan, M. K. et al., J. Gen. Virol. 78: 2813-2820, 1997; Sakai, Y. et al., FEBS Lett. 456: 221-226, 1999). The present inventors performed gene transfer into a kidney using the recombinant Sendai virus vector to determine whether or not the use of the vector could overcome the problems of the previous gene transfer vectors. Specifically, the inventors studied *in-vivo* transfection activity of Sendai virus vector, including transfection efficiency, persistency and efficiency of gene expression, and effect of vector administration route, from the clinical standpoit.

As a result, a recombinant Sendai virus vector comprising exogenous genes was revealed to be able to transfer genes into renal cells with high efficiency *in vivo.* With either administration method of perfusion from the renal artery or of backflow from the urinary tract, the vector could transfer genes into renal cells diffusively over a wide area of kidney, and in particular, the administration from the renal artery resulted in high-level expression of GFP in cells such as interstitial cells and glomerulus cells. The persistent expression of the gene transferred via Sendai virus could be observed for at least about 1 month. In addition, in kidney tissues that were kept for a long period of time, the expression of the transgene was also detectable in renal tubular cells. On the other hand, in the administration to cause backflow from the urinary tract, the transgene was expressed mainly in renal tubular cells (distal and proximal renal tubules), and also expressed in interstitial cells.

Specifically, the present inventors have found: (1) that the recombinant Paramyxovirus vector can express a gene in renal cells with high efficiency; (2) that the vector allows to introduce a gene into different types of cells depending on the administration route and the administration from the renal artery results in no macrophage infiltration to regions in proximity to interstitial cells and glomerular tissues; and (3) that the viral genome and the exogenous gene are expressed stably for long periods. These findings demonstrate that the Paramyxoviral vector is useful for gene transfer into kidney. The applicability of adenoviral vectors is currently tested in the field of gene transfer into kidney. For the purpose of gene transfer into kidney, the Paramyxoviral vector of the present invention can be a vector that has the compatibility equivalent to or higher than that of adenovirus. The recombinant Paramyxovirus vectors as well as adenoviral vectors have several important advantages such that both high level expression of transgenes and high gene transfer efficiency do not depend on the cell cycle.

A problem often associated with adenoviral vectors is that sufficient infectivity to target cells is not achieved by short time infection. This may be because long contact is required to achieve incorporation of vector particles into cells *via* Coxsackie-adenovirus receptor (CAR). In *in-vivo* administration to kidney, long-term blocking of bloodstream during administration results in tissue destruction due to ischemic injury. Thus, such long-term bloodstream blocking is unfavorable. In contrast, Paramyxovirus exhibits sufficiently high infectivity to cells with short-time exposure, and thus is highly advantageous in clinical gene transfer. Because of such benefits of Paramyxovirus vector, the Paramyxoviral vector of the present invention can be a very excellent vector for gene therapy in various clinical cases.

Specifically, the present invention relates to a Paramyxoviral vector for gene transfer to renal cells and a gene transfer method using the vector, and more specifically, to:
(1) a method of introducing a gene into a renal cell, wherein the method comprises contacting a Paramyxovirus vector with the renal cell;
(2) the method according to (1), wherein the method further comprises administering the Paramyxovirus vector into a blood vessel;
(3) the method according to (2), wherein the blood vessel is renal artery;
(4) the method according to (1), wherein the method further comprises administering the Paramyxovirus vector into urinary tract;
(5) the method according to any one of (1) to (4), wherein the renal cell is selected from the group consisting of an interstitial cell, a glomerular cell and a renal tubular cell;
(6) the method according to any one of (1) to (5), wherein the Paramyxovirus is Sendai virus;
(7) a Paramyxovirus vector for gene transfer into a renal cell;
(8) a composition for gene transfer into a renal cell, wherein the composition comprises a Paramyxovirus vector or a cell comprising the vector;
(9) the composition according to (8), wherein the composition is administered into a blood vessel;
(10) the composition according to (9), wherein the blood vessel is renal artery;
(11) the composition according to (8), wherein the composition is administered into urinary tract;
(12) the composition according to any one of (8) to (11), wherein the renal cell is selected from the group consisting of an interstitial cell, a glomerular cell and a renal tubular cell; and
(13) the composition according to any one of (8) to (12), wherein the Paramyxovirus is Sendai virus.

Herein, a "Paramyxovirus vector" is defined as a vector (or carrier) that is derived from the Paramyxovirus and that is used for gene transfer to host cells. The Paramyxovirus vector of the present invention may be ribonucleoprotein (RNP) or a virus particle having infectivity. Here, "infectivity" is defined as the ability of the recombinant Paramyxovirus vector to transfer, through its cell adhesion and membrane fusion abilities, a gene contained in the vector to cells to which the vector is adhered. In a preferred embodiment, the Paramyxovirus vector of the present invention carries an exogenous gene in an expressible manner. The Paramyxovirus vector may have a replication ability, or may be a defective vector without the replication ability. Herein, "replication ability" is defined as the ability of virus vectors to replicate and produce infective virus particles in host cells infected with the virus vectors.

The term "recombinant" product refers to a compound or composition produced *via* a recombinant polynucleotide. The recombinant polynucleotide refers to a polynucleotide in which nucleic acids are not linked in a non-naturally occurring manner. Herein, a "recombinant" Paramyxovirus vector is defined as a Paramyxovirus vector constructed by gene engineering or its amplified products. For instance, recombinant Paramyxovirus vectors can be generated by reconstitution of a recombinant Paramyxovirus cDNA.

Herein, a Paramyxovirus is defined as a virus of the *Paramyxoviridae* family or a derivative thereof. Paramyxoviruses used in the present invention include, for example, viruses belonging to the *Paramyxoviridae* such as Sendai virus, Newcastle disease virus, Mumps virus, Measles virus, Respiratory syncytial virus, rinderpest virus, distemper virus, simian parainfluenza virus (SV5), and type I, II, and III human parainfluenza virus. The virus of the present invention may be preferably a virus of the genus *Paramyxovirus* or a derivative thereof . Paramyxoviruses that can be used in the present invention includes, for example, type I human parainfluenza virus (HPIV-1), type III human parainfluenza virus (HPIV-3), type III bovine parainfluenza virus (BPIV-3), Sendai virus (also referred to as "type I mouse parainfluenza virus"), and type X simian parainfluenza virus (SPIV-10). Most preferably, the Paramyxovirus of the invention may be the Sendai virus. These viruses may be naturally-occurring, wild-type, mutant, laboratory-passaged strains, artificially constructed strains, etc. Incomplete viruses such as the DI particle (Willenbrink W. and Neubert W. J., J. Virol., 1994, 68, 8413-8417), synthesized oligonucleotides, and so on, may also be utilized as a material for generating the virus vector of the present invention.

Genes encoding proteins of a Paramyxovirus include NP, P, M, F, HN, and L genes. Here, the "NP, P, M, F, HN, and L genes" represent those encoding the nucleocapsid protein, phosphoprotein, matrix protein, fusion protein, hemagglutinin-neuraminidase, and large protein, respectively. Genes of each virus of the subfamily Paramyxovirus are described generally as follows. In general, NP gene may also be indicated as "N gene".

| | | | | | | | |
|---|---|---|---|---|---|---|---|
| Paramyxovirus | NP | P/C/V | M | F | HN | - | L |
| Rublavirus | NP | P/V | M | F | HN | (SH) | L |
| Morbillivirus | NP | P/C/V | M | F | H | - | L |

For instance, the accession numbers of each gene of the Sendai virus classified as a Respirovirus of *Paramyxoviridae* in the nucleotide sequence database, are M29343, M30202, M30203, M30204, M51331, M55565, M69046, and X17218 for NP gene; M30202, M30203, M30204, M55565, M69046, X00583, X17007, and X17008 for Pgene; D11446, K02742, M30202, M30203, M30204, M69046, U31956, X00584, X53056 for M gene; D00152, D11446, D17334, D17335, M30202, M30203, M30204, M69046, X00152, and X02131 for F gene; D26475, M12397, M30202, M30203, M30204, M69046, X00586, X02808, X56131 for HN gene; and D00053, M30202, M30203, M30204, M69040, X00587, and X58886 for L gene.

As used herein, the term "gene" refers to a genetic substance, including nucleic acids such as RNA and DNA. A gene may or may not encode a protein. For example, a gene may encode a functional RNA such as ribozyme or antisense RNA. A gene can be a naturally-occurring sequence or an artificially designed sequence. Furthermore, as used herein, the term "DNA" includes a single-stranded DNA and a double-stranded DNA.

The term "renal cell" refers to a cell in normal mammalian kidney and a cell infiltrating in diseased kidney. Such cells infiltrating into kidney include, for example, monocytes, macrophages and leukocytes.

Specifically, mammalian kidney comprises at least 15 types of cells, including the following:
glomerular cell;
mesangial cell;
interstitial cell;
renal tubular cell;
podocyte; and
endothelial cell.

The glomerular basement membrane (GBM) and epithelial cell are also included. In the present invention, these cells are included in the renal cells.

The renal artery refers to arteries inside and towards a kidney, including renal arteries branched from the abdominal aorta and the downstream interlobular arteries, right renal arteries, and left renal arteries; arteries branching off near the hilum renalis; five segmental arteries and the downstream arcuate arteries. The renal vein refers to veins inside and towards a kidney, including right renal veins, left renal veins, interlobular veins, stellate veins, arcuate veins, and interlobar veins.

The present invention provides the use of Paramyxovirus vector for gene transfer into renal cells. The present inventors found that Paramyxovirus could allow introduction of genes into renal cells with high efficiency. The present inventors obtained the experimental results showing that the expression of transgenes was detected in a wide area in the kidney. In particular, when injected from the renal artery, the genes were confirmed to be transferred preferentially into interstitial cells and glomerular cells. These cells are important as target cells in renal diseases, and thus the vector of the present invention can be used suitably for gene therapy for renal diseases.

In addition, the present inventors revealed that the genes transferred into renal cells using a recombinant Paramyxovirus were persistently expressed at least over one month. This shows an advantage that continuous therapeutic effect can be obtained when gene therapies targeted at renal cells was performed using a recombinant Paramyxovirus vector.

Paramyxovirus vectors can be preferably utilized in clinical trials of human gene therapy in view of safety as well. First, it is a major obstacle in high efficient gene transfer that introduced DNA must be transported into the nucleus for the expression of an exogenous gene. In the case of Sendai virus and such, however, expression of an exogenous gene is driven by both cellular tubulin and its RNA polymerase (L protein) in the cytoplasm. This suggests that the Sendai virus does not interact with the genome of host cells, which avoids risks such as tumorigenesis. Second, the Sendai virus is known to be pathogenic in rodents causing pneumonia, but not in humans, which is supported by studies showing that the intranasal administration of the wild type Sendai virus does not do harm in nonhuman primates (Hurwitz J. L. et al., Vaccine, 1997, 15, 533-540). These features suggest that Sendai virus vector can be utilized in human therapy, and further, support the notion that Sendai virus can be one of the promising alternatives in gene therapy to renal cells.

Thus, the finding obtained by the present invention that Paramyxovirus vector has various advantages in gene transfer into renal cells may bring great advance in gene therapy, especially those targeted at renal cells.

The recombinant Paramyxovirus vector of the present invention used for gene transfer into renal cells is not limited to any special kind. For instance, suitable Paramyxovirus vectors include vectors that are able to replicate and autonomously propagate. In general, the genome of the wild type Paramyxovirus contains a short 3' leader region followed by six genes encoding N (nucleocapsid), P (phospho), M (matrix), F (fusion), HN (hemagglutinin-neuraminidase), and L (large) proteins, and has a short 5' trailer region on the other terminus. The vector of the present invention that is able to replicate autonomously can be obtained by designing a genome having a similar structure to that described above. In addition, a vector for expressing an exogenous gene can be obtained by inserting the exogenous gene to the genome of the above vector. The Paramyxovirus vector of the invention may have an altered alignment of virus genes, compared with wild type virus.

The Paramyxovirus vector of the invention may have deletion(s) of some of the genes that are contained in the wild type virus. For instance, when the Sendai virus vector is reconstituted, proteins encoded by NP, P/C, and L genes are thought to be required in trans, but the genes may not be a component of the virus vector. In one embodiment, an expression vector carrying genes encoding the proteins may be co-transfected into host cells with another expression vector encoding the vector genome to reconstitute a virus vector. Alternatively, an expression vector encoding the virus genome is transfected into host cells carrying genes encoding the proteins, and thus a virus vector can be reconstituted by using the proteins provided by the host cell. The amino acid sequence of these proteins may not be identical to those derived from the original virus as long as it has an equivalent or higher activity in nucleic acid transfer, and may be mutated or replaced with that of a homologous gene of another virus.

Proteins encoded by M, F, and HN genes are thought to be essential for cell-to-cell propagation of a Paramyxovirus vector. However, these proteins are not required when the vector is prepared as RNP. If genes M, F, and HN are components of the genome contained in RNP, products of these genes are produced when introduced into host cells, and virus particles having infectivity are generated. RNP vectors that produce an infective virus include a virus genomic RNA encoding N, P, M, F, HN, and L genes and RNP containing N, P, and L proteins. When such RNP is introduced into cells, virus genome is expressed and replicated through functions of the proteins, and thus infective virus vectors are amplified.

RNP can be introduced into cells as a complex with a transfection reagent such as lipofectamine and polycationic liposome. Specifically, a variety of transfection reagents can be used, for instance, DOTMA (Boehringer), Superfect (QIAGEN #301305), DOTAP, DOPE, DOSPER (Boehringer #1811169). Chloroquine may be added to prevent degradation in the endosome (Calos M. P., Proc. Natl. Acad. Sci. USA, 1983, 80, 3015). In the case of replicative viruses, the produced viruses can be amplified or passaged by re-infecting into cultured cells, chicken eggs, or animals (e.g. mammalian such as mice).

Vectors lacking the M, F, and/or HN genes are also used as the Paramyxovirus vector of the present invention. These vectors can be reconstituted by providing deleted gene products exogenously. Such vectors can still adhere to host cells and induce cell fusion like the wild type. However, daughter virus particles do not have the same infectivity as the original ones because the vector genome introduced into cells lacks one of the above genes. Therefore, these vectors can be useful as safe virus vectors that are capable of only a single gene transfer. For instance, genes deleted from the genome may be F and/or HN genes. Virus vectors can be reconstituted by co-transfection of an expression plasmid encoding the genome of a recombinant Paramyxovirus lacking the F gene, an expression vector for the F protein, and that for NP, P/C, and L proteins into host cells (International Publication numbers WO00/70055 and WO00/70070). Alternatively, host cells in which the F gene is integrated into the chromosome may be used. The amino acid sequence of these proteins provided exogenously may not be identical to those of the wild type and may be mutated or replaced by a homologous protein of another virus as long as they provide equivalent or higher gene transfer activity.

The envelope protein of the Paramyxovirus vector of the invention may contain another protein than the envelope protein of the original vector genome. There is no limitation on such proteins. These include envelope proteins of other viruses such as the G protein of the vesicular stomatitis virus (VSV-G). Thus, the Paramyxovirus vector of the invention includes a pseudo type virus vector that has an envelope protein derived from a virus different from the original virus.

The Paramyxoviral vector of the present invention may also comprise, for example, on the viral envelop surface, proteins capable of adhering to particular cells, such as adhesion factors, ligands and receptors or chimeric proteins comprisinga protein described above on the outer surface and viral envelop-derived polypeptides inside the virus. It enables the production of a vector targeting a particular tissue. These proteins may be encoded by the virus genome itself, or supplied at the time of virus reconstitution through expression of genes other than virus genome (for example, genes derived from another expression vector or host cell chromosome).

The virus genes contained in the vector of the present invention may be altered, for example, to reduce antigenicity or enhance RNA transcription efficiency or replication efficiency. Specifically, it is possible to alter at least one of the NP, P/C, and L genes, which are genes of replication factors, to enhance transcription or replication. It is also possible to alter the HN protein, a structural protein having hemagglutinin activity and neuraminidase activity, to enhance the virus stability in blood by weakening the former activity and to regulate infectivity by altering the latter activity. It is also possible to alter the F protein, which is implicated in membrane fusion, to regulate the fusion ability of membrane-fused liposomes. Furthermore, it is possible to analyze the antigen presenting epitopes and such of possible antigenic molecules on the cell surface such as the F protein and HN protein and use them to generate a Paramyxovirus that is engineered to have weak antigen presenting ability.

When Sendai virus is used as a vaccine of the present invention, it maylack accessory genes. For example, the disruption of the V gene, one of the accessory genes of Sendai virus, results in reduction of pathogenicity of Sendai virus toward hosts such as mouse without affecting gene expression and replication in cultured cells (Kato, A. et al. 1997. J. Virol. 71:7266-7272; Kato, A. et al. 1997. EMBO J. 16:578-587; Curran, J. et al., WO 01/04272, EP 1067179). Such attenuated vectors are particularly suitable as vectors constituting vaccines of the present invention.

A viral vector of the present invention can encode exogenous genes in its genomic RNA. A recombinant Paramyxovirus vector comprising exogenous genes can be prepared by inserting exogenous genes into the above-mentioned Paramyxovirus vector genome. The exogenous gene can be a desired gene to be expressed in target renal cells. The exogenous gene may encode a naturally occurring protein, or a modified protein prepared by modifying the original protein by deletion, substitution or insertion, as long as the modified protein isfunctionally equivalent to the naturally occurring protein. Such a gene may also encode a deletion protein derived from the naturally occurring protein, or an artificially designed protein. For instance, for the purpose of gene therapy and such, a gene used to treat a target disease may be inserted into the DNA encoding the genome of the virus vector. In the case of inserting an exogenous gene into Sendai virus vector DNA, a sequence comprising nucleotides of multiples of six is desirably inserted between the transcription end sequence (E) and the transcription start sequence (S) (Calain P. and Roux L., J. Virol., 1993, 67(8), 4822-4830). An exogenous gene can be inserted upstream and/or downstream of each of the virus genes (NP, P, M, F, HN, and L genes). In order not to interfere with the expression of upstream and downstream genes, an E-I-S sequence (transcription end sequence-intervening sequence-transcription start sequence) or a portion of it may be suitably placed upstream or downstream of an exogenous gene so that E-I-S sequence is located between each gene. Alternatively, an exogenous gene can be inserted *via* IRES sequence.

Expression level of inserted exogenous genes can be regulated by the type of transcription start sequence that is attached to the upstream of the genes. It also can be regulated by the position of insertion and the sequence surrounding the gene. In the Sendai virus, for instance, the closer to the 3'-terminus of the negative strand RNA of the virus genome (the closer to NP gene in the gene arrangement on the wild type virus genome) the insertion position is, the higher the expression level of the inserted gene will be. To achieve a high expression of an exogenous gene, it is preferably inserted into the upstream region of the negative stranded genome such as the upstream of the NP gene (3'flanking sequence on the minus strand), or between NP and P genes. Conversely, the closer to the 5'-terminus of the negative strand RNA (the closer to L gene in the gene arrangement on the wild type virus genome) the insertion position is, the lower the expression level of the inserted gene will be. To reduce the expression of an exogenous gene, it may be inserted into the most 5' position on the negative strand, that is, downstream of the L gene in the wild type virus genome (5' flanking region of the L gene on the negative strand) or upstream of the L gene (3' flanking region of L gene on the negative strand) . Thus, the insertion position of an exogenous gene can be properly adjusted so as to obtain a desired expression level of the gene or optimize the combination of the insert with the virus genes surrounding it. For instance, if the overexpression of a gene introduced by a high titer virus vector may cause toxicity, it is possible not only to control the virus titer, but also to reduce the expression level of individual Sendai virus vectors by designing the insertion position closer to the 5'-terminus of the negative strand, or replacing the transcription start sequence with one having lower efficiency so as to obtain an appropriate effect.

In general, high-level expression of an antigen protein is favorable for the immunity, as long as the protein exhibits no cytotoxicity. Therefore, it is preferable to connect the gene encoding an antigen protein to a transcription initiation sequence of high transcription efficiency and insert it near the 3' end of the negative strand genome. Examples of suitable vectors are those in which an exogenous gene is placed downstream of all of the viral proteins of the Paramyxovirus in the negative strand genome of a Paramyxovirus vector. For example, a preferred vector has an exogenous gene upstream of the N gene (on the 3' side in the negative strand). Alternatively, an exogenous gene may be inserted immediately downstream of the N gene.

To help the easy insertion of an exogenous gene, a cloning site may be designed at the position of insertion. For example, the cloning site may be the recognition sequence of restriction enzymes. The restriction sites in the virus vector DNA can be used to insert an exogenous gene. The cloning site may be a multicloning site that contains recognition sequences for multiple restriction enzymes. The vector of the present invention may have other exogenous genes at positions other than that used for above insertion.

Construction of a recombinant Sendai virus vector having an exogenous gene can be performed as follows, for example, according to the method described in Hasan, M. K. et al., J. Gen. Virol., 1997, 78: 2813-2820, Kato A. et al., EMBO J., 1997, 16: 578-587, and Yu D. et al., Genes Cells, 1997, 2: 457-466.

First, a DNA sample containing a cDNA sequence encoding a desired exogenous gene is prepared. It is preferable that the concentration of the sample is 25 ng/µl or higher and that it can be detected as a single plasmid by electrophoresis. The following description is an example where an exogenous gene is inserted into the NotI site of virus genomic DNA. If the cDNA sequence contains a NotI site, the site is desirably removed in advance by altering the nucleotide sequence using the known method such as site-directed mutagenesis while maintaining the encoded amino acid sequence. A desired DNA fragment is amplified by PCR from the DNA sample. In order to obtain a fragment having NotI sites at both ends and to add a single copy of the transcription end sequence (E), intervening sequence (I) , and transcription start sequence (S) of the Sendai virus (EIS sequence) to one end, synthesized DNA sequences (primer pair), namely, a pair of a forward primer (sense strand) comprising a part of the desired gene, and a reverse primer (antisense) comprising a NotI recognition site, E, I, and S sequences, and part of the desired gene, is prepared.

For example, the forward synthetic DNA sequence contains two or more nucleotides at the 5'-terminus to ensure digestion with NotI (preferably 4 nucleotides not containing a sequence derived from the NotI recognition site, such as GCG and GCC; more preferably ACTT). To the 3'-terminus of the sequence, the NotI recognition sequence GCGGCCGC is added. Furthermore, to the 3'-terminus, as a spacer, any 9 nucleotides or those of 9 plus multiples of 6 are added. Furthermore, to the 3'-terminus, a sequence of approximately 25 nucleotides corresponding to the ORF of the desired cDNA starting from the initiation codon ATG is added. The 3'-terminus of the forward synthetic oligo DNA containing approximately 25 nucleotides of the desired cDNA is preferably selected so that the last nucleotide is G or C.

The reverse synthetic DNA sequence contains two or more nucleotides at the 5'-terminus (preferably 4 nucleotides not containing a sequence derived from the NotI recognition site, such as GCG and GCC; more preferably ACTT). To the 3'-terminus of the sequence, the NotI recognition sequence GCGGCCGC is added. Furthermore, to the 3'-terminus, a spacer oligo DNA is added to adjust the length of the primer. The length of the oligo DNA is designed so that it is a multiple of 6 nucleotides including the NotI recognition sequence GCGGCCGC, the sequence complementary to the cDNA, and the EIS sequence derived from the Sendai virus genome as described below (so-called "rule of six"; Kolakofski D. et al., J. Virol., 1998, 72, 891-899; Calain P. and Roux L., J. Virol., 1993, 67, 4822-4830). Furthermore, to the 3'-terminus of the added sequence, complementary sequences to the S sequence of the Sendai virus, preferably 5'-CTTTCACCCT-3' (SEQ ID NO: 1), to the I sequence, preferably 5'-AAG-3', and to the E sequence, preferably 5'-TTTTTCTTACTACGG-3' (SEQ ID NO: 2) are added. Finally, to the 3'-terminus, a sequence, which is selected so that the last nucleotide of the complementary sequence of the desired cDNA becomes G or C, is added, where the last nucleotide is approximately 25 nucleotides upstream from the termination codon. Thus, the 3'-teminus of the reverse synthetic oligo DNA is prepared.

PCR can be performed by a common method using, for example, ExTaq polymerase (TaKaRa). Vent polymerase (NEB) may be used preferably, and the amplified fragment is digested with NotI, and inserted into the NotI site of the plasmid vector pBluescript. The nucleotide sequence of the obtained PCR product is checked with an automated DNA sequencer, and a plasmid having the correct sequence is selected. The insert is excised from the plasmid by NotI digestion, and subcloned into the NotI site of the plasmid comprising Paramyxovirus genomic cDNA. Alternatively, the PCR products may be directly cloned into the NotI site without using pBluescript vector to obtain recombinant Paramyxovirus virus cDNA.

For example, recombinant Sendai virus genomic cDNA can be constructed according to the methods described in Yu, D. et al., Genes Cells, 1997, 2, 457-466 or Hasan M. K. et al., J. Gen. Virol., 1997, 78, 2813-2820. For example, a spacer sequence of 18 bp containing the NotI site (5'- (G)-CGGCCGCAGATCTTCACG-3'; SEQ ID NO: 3) is inserted into an adjacent gene locus of a cloned Sendai virus genomic cDNA (pSeV(+)) between the leader sequence and the 5'-terminus of a sequence encoding the N protein, and the plasmid pSeV18⁺b(+) containing a self-cleavable ribozyme site derived from the antigenomic strand of the hepatitis delta virus is obtained (Hasan M. K. et al., J. General Virol., 1997, 78, 2813-2820). An exogenous gene fragment is inserted into the NotI site of pSeV18⁺b(+) to obtain a recombinant Sendai virus cDNA into which a desired exogenous gene has been inserted.

A viral genome-encoding DNA is ligated with an appropriate transcriptional promoter to construct a vector DNA. The resulting vector is transcribed *in vitro* or in cells, and RNP is reconstituted in the presence of Paramyxoviral L, P and NP proteinsto produce a viral vector comprising the RNP. The present invention provides a method of producing a Paramyxovirus vector for introducing a gene into renal cells, or a method of producing a composition for gene transfer into renal cells comprising the vector, wherein the methods comprise transcribing the Paramyxovirus vector genomic DNA. The present invention also provides such viral-genome-encoding DNA for producing a Paramyxovirus vector, wherein said DNA is used as a component of the Paramyxoviral vector of the present invention or a composition for gene transfer into renal cells comprising the vector. The present invention also relates to the use of DNA encoding the vector genome, for producing the Paramyxovirus vector of the present invention or the Paramyxovirus vector that is a component of the composition for gene transfer into renal cells comprising the vector. Reconstitution of a virus from virus vector DNA can be performed according to the known methods (WO97/16539; WO97/16538; Durbin A. P. et al., Virol., 1997, 235, 323-332; Whelan S. P. et al., Proc. Natl. Acad. Sci. USA, 1995, 92, 8388-8392; Schnell M. J. et al., EMBO J., 1994, 13, 4195-4203; Radecke F. et al., EMBO J., 1995, 14, 5773-5784; Lawson N. D. et al., Proc. Natl. Acad. Sci. USA, 1995, 92, 4477-4481; Garcin D. et al., EMBO J., 1995, 14, 6087-6094; Kato A. et al., Genes Cells, 1996, 1, 569-579; Baron M. D. and Barrett T., J. Virol., 1997, 71, 1265-1271; Bridgen A. and Elliott R. M., Proc. Natl. Acad. Sci. USA, 1996, 93, 15400-15404). These methods enable the reconstitution of Paramyxovirus vectors including the parainfluenza virus, vesicular stomatitis virus, rabies virus, measles virus, rinderpest virus, and Sendai virus vectors from DNA. If the F, HN, and/or M genes are deleted from the virus vector DNA, infective virus particles will not be formed. However, it is possible to generate infective virus particles by introducing these deleted genes and/or genes encoding an envelope protein from another virus into the host cells and expressing them.

Methods for introducing vector DNA into cells may include (1) forming DNA precipitates that can be incorporated into desired cells, (2) making a complex that comprises positively charged DNA, that is suitable for being incorporated into desired cells and that has low cytotoxicity, and (3) instantaneously opening a pore large enough for DNA to pass through in the desired plasma membrane using an electrical pulse.

A variety of transfection reagents can be used in (2), for instance, DOTMA (Boehringer), Superfect (QIAGEN #301305), DOTAP, DOPE, and DOSPER (Boehringer #1811169). For (1), transfection using calcium phosphate can be used. In this method, DNA incorporated by cells is taken up into phagocytic vesicles, but it is known that a sufficient amount of DNA is also taken up into the nucleus (Graham F. L. and van Der Eb J., Virol., 1973, 52, 456; Wigler M. and Silverstein S., Cell, 1977, 11, 223). Chen and Okayama studied the optimization of the transfer technology and reported (1) that maximal efficiency is obtained when cells and precipitates are incubated under 2 to 4% CO₂ at 35°C for 15 to 24 hr, (2) that circular DNA has higher activity than linear DNA, and (3) that the optimal precipitates are formed when the DNA concentration in the mixed solution is 20 to 30 µg/ml (Chen C. and Okayama H., Mol. Cell. Biol., 1987, 7, 2745). The method of (2) is suitable for transient transfection. More classically, a transfection method in which DEAE-dextran (Sigma #D-9885 M. W. 5 x 10⁵) is mixed with DNA at a desired concentration ratio is known. Because most complexes are degraded in the endosome, chloroquine may be added to enhance the transfection efficiency (Calos M. P., Proc. Natl. Acad. Sci. USA, 1983, 80, 3015). The method of (3), called electroporation, may be more broadly applied than the methods of (1) and (2) because it can be used for any kind of cells. The transfection efficiency can be maximized by optimizing the duration of pulse currents, the form of pulse, the strength of the electrical field (gap between electrodes, and voltage), conductivity of buffer, DNA concentration, and cell density.

Among the above three methods, the method of (2) is suitable for introducing DNA into cells to reconstitute the vector because it is easy to perform and enables the testing of a large number of samples using a large amount of cells. Preferable transfection reagents include, the Superfect Transfection Reagent (QIAGEN, #301305) and the DOSPER Liposomal Transfection Reagent (Boehringer Mannheim #1811169), but are not limited thereto.

Specifically, the reconstitution from cDNA are performed as follows.

LLC-MK2, a cell line derived from a monkey kidney, is cultured in a 24-well to 6-well plastic plate or in a 100-mm petri dish in minimum essential medium (MEM) containing 10% fetal calf serum (FCS) and an antibiotic (100 units/ml penicillin G and 100 µg/ml streptomycin) to be 70 to 80% confluent. Cells are then infected, for instance, at 2 pfu/cell with recombinant vaccinia virus vTF7-3 that expresses T7 polymerase, which has been inactivated by a 20-minute UV exposure in the presence of 1 µg/ml psoralen (Fuerst T. R. et al., Proc. Natl. Acad. Sci. USA, 1986, 83, 8122-8126; and Kato. A. et al., Genes Cells, 1996, 1, 569-579). The amount of psoralen and the duration of UV exposure can be optimized. One hour after infection, cells are transfected by, for example, lipofection using Superfect (QIAGEN) with 2 to 60 µg of, or more preferably 3 to 5 µg of the above recombinant Sendai virus cDNA together with expression plasmids for virus proteins (24-0.5 µg pGEM-N, 12-0.25 µg pGEM-P, and 24-0.5 µg pGEM-L, or more preferably 1 µg pGEM-N, 0.5 µg pGEM-P, and 1 µg pGEM-L) (Kato. A. et al., Genes Cells, 1996, 1, 569-579) that function in trans and are required for producing a full length Sendai virus genome. The transfected cells are cultured in serum free MEM containing, if desired, 100 µg/ml rifampicin (Sigma) and cytosine arabinoside (AraC) (Sigma) whose concentration is more preferably 40 µg/ml, so that the drug concentration is adjusted to be optimal to minimize the cytotoxicity of the vaccinia virus and maximize the recovery of virus (Kato. A. et al., Genes Cells, 1996, 1, 569-579). Cells are cultured for 48 to 72 hr after transfection, then collected and lysed through three cycles of freeze-thawing. The cell lysates are transfected into LLC-MK2 cells, and after a 3- to 7-day culture, the culture medium is collected. To reconstitute a virus vector lacking a gene encoding an envelope protein that is incapable of replication, the vector may be transfected into LLC-MK2 cells expressing an envelope protein, or co-transfected with expression plasmid for the envelope protein. Alternatively, transfected cells can be overlaid and cultured on LLC-MK2 cells expressing envelope protein to propagate a deletion virus vector (International Publication Numbers WOO/70055 and WOO/70070). Alternatively, the above cell lysates prepared by freeze-thawing can be innoculated into the allantoic membrane of an embryonated chicken egg of ten days to collect the allantoic fluidsafter about three days. The virus titer of the culture medium or the allantoic fluidscan be determined by measuring hemagglutinin activity (HA). The HA may be determined by "endo-point dilution" (Kato. A. et al., Genes Cells, 1996, 1, 569-579; Yonemitsu Y. and Kaneda Y., Hemagglutinating virus of Japan-liposome-mediated gene delivery to vascular cells., Molecular Biology of Vascular Diseases. Methods in Molecular Medicine, Ed. by Baker A. H., Humana Press, 1999, 295-306). To eliminate the possible contamination of vaccinia virus vTF7-3, the obtained allantoic fluid sample may be diluted appropriately (10⁶ times for instance) and re-amplified in chicken eggs. Re-amplification may be repeated, for example, three times or more. The obtained virus stock can be stored at -80°C.

The collected Paramyxovirus can be purified so as to be substantially pure. Purification can be carried out by a known purification/separation method such as filtration, centrifugation and column purification, or the combination thereof. The term "substantially pure" means that an isolated substance (compound, polypeptide, virus, etc.) comprises the major portion in a sample where it is present as a component. Typically, a substantially pure component in a sample comprises 50% or more, preferably 70% or more, more preferably 80% or more, yet more preferably 90% or more, of the total amount including the other components in the sample. Such a percentage can be estimated by a procedure known to those skilled in the art, for example, in weight ratio [w/w]. Those of solvent, salts, additive compounds and so on should be excluded to calculate the ratio. Exemplary purification methods specific for Paramyxovirus include methods using cellulose sulfuric ester or cross-linked polysaccharide sulfuric ester (Examined Published Japanese Patent Application No. (JP-B) Sho 62-30752; JP-B Sho 62-33879; JP-B Sho 62-30753), and methods which comprise allowingpolysaccharide comprising fucose sulphuric acid and/or its degradation productto adsorb the virus (WO97/32010).

Host cells are not limited to any special types of cells as long as the virus vector can be reconstituted in the cells. Host cells may include LLC-MK2 cells, CV-1 cells derived from a monkey kidney, cultured cell lines such as BHK cells derived from a hamster kidney, and human-derived cells. Furthermore, to obtain a large quantity of the Sendai virus vector, embryonated chicken eggs may be infected with virus vectors obtained from the above host cells and the vectors can be amplified. The method of producing virus vectors using chicken eggs has been established (Advanced protocols in neuroscience study III, Molecular physiology in neuroscience., Ed. by Nakanishi et al., Kouseisha, Osaka, 1993, 153-172). Specifically, for example, fertilized eggs are incubated for 9 to 12 days at 37 to 38°C in an incubator to grow the embryos. Virus vectors are inoculated into the allantoic cavity, and eggs are further incubated for several days to propagate the vectors. Conditions such as the duration of incubation may vary depending on the type of recombinant Sendai virus used. Then, the allantoic fluidscontaining viruses is recovered. Sendai virus vector is separated and purified from the allantoic fluid sample according to the standard method (Tashiro M., Protocols in virus experiments., Ed. by Nagai and Ishihama, MEDICAL VIEW, 1995, 68-73).

In preparing deletion virus vectors, two different virus vectors having deletion of a different envelope gene in the virus vector genome may be transfected into the same cell. In this case, each deleted envelope protein is supplied through expression from the other vector, and this mutual complementation permits the generation of infective virus particles, which can replicate and propagate. Thus, two or more of the virus vectors of the present invention may be simultaneously inoculated in a combination that complement each other, thereby producing a mixture of each envelope deletion virus vector at a low cost and in a large scale. Because these viruses lacking an envelope gene have a smaller genome, they can allow the insertion of a long exogenous gene. In addition, it is difficult for these viruses, which are intrinsically non-infective, to keep the status of co-infection after being diluted outside cells, and thus they are sterilized and less harmful to the environment.

Once a viral vector is prepared using, as the exogenous gene, a gene for the treatment of a disease, then the vector can be administered to perform gene therapy. When the viral vector of the present invention is used in gene therapy, an exogenous gene that ensures desired therapeutic effects or an endogenous gene whose expression is impaired in the body of a patient can be expressed either by a method of direct administration or by a method of indirect (*ex vivo*) administration for gene expression. There is no limitation on the type of exogenous gene, including not only a nucleic acid encoding a protein but also a nucleic acid encoding no protein, for example, antisense or ribozyme.

The Paramyxovirus vector of the present invention can be made as a composition for gene transfer into renal cells together with a desired, pharmaceutically acceptable carrier. Herein, a "pharmaceutically acceptable carrier" is defined as those materials that can be administered with a vector and does not significantly inhibit gene transfer achieved by the vector. For instance, the Paramyxovirus vector of the invention may be appropriately diluted with saline, phosphate buffered saline (PBS), and so on, to make a composition. If the Paramyxovirus vector of the invention is propagated in chicken eggs, the composition may contain allantoic fluids. Also, the composition may contain carriers or media such as deionized water or a 5% dextrose aqueous solution. It may further contain plant oils, suspensions, detergents, stabilizers, antibiotics, etc. Furthermore, preservatives and other additives can also be added in the composition.

An exogenous gene which is carried by the Paramyxovirus vector can be transferred into renal cells, by contacting renal cells with the Paramyxovirus vector obtained as described above or a composition comprising the vector. The present invention provides the use of the Paramyxovirus vector to introduce a gene into renal cells. The type of infection route is not limited and include, for example, systemic administration by intravenous injection or such andtopical administration to restrict the area where the vector is introduced. The topical administration to kidney can be achieved by administration into kidney blood vessels, urinary tract, renal pelvis, parenchyma of kidney, etc. Specifically, preferred route of the administration include injection from the renal artery or vein and injection from the urinary tract or renal pelvis to cause backflow (Tomita, N. et al., Biochem. Biophys. Res. Commun. 186: 129-134, 1992; Isaka, Y. et al., J. Clin. Invest. 92: 2597-2601; Arai, M. et al., Biochem. Biophys. Res. Commun. 206: 525-532, 1995; Heikkila, P. et al., Gene Ther. 3: 21-27, 1996; Rappaport, J. et al., Kidney Int. 47: 1462-1469, 1995; Oberbauer, R. et al., Kidney Int. 48: 1226-1232, 1995; Haller, H. et al., Kidney Int. 50: 473-480, 1996; Lien, Y. et al., Exp. Nephrol. 5: 132-136; Moullier, P. et al., Kidney Int. 45: 1220-1225, 1994; Bosch, R. et al., Exp. Nephrol. 1: 49-54, 1993; Zhu, G. et al., Gene Ther. 3: 298-304). In particular, injection of the vector *via* blood vessels is preferred becasue such injection may result in gene transfer into interstitial cells and glomerulus with high efficiency and induce no infiltration of macrophages near these cells.

In injection *via* a blood vessel, for example, a vector comprising a gene of interest may be injected into an artery *via* a catheter (see, U.S. patent No. 5,328,470) or a winged infusion set (Terumo Medical Corporation). It is preferable to block the blood stream with a clamp or such during injection. For example, a catheter is inserted into the left renal artery, the artery is clamped near the abdominal aorta to block the blood stream, and after perfusion with saline, a vector solution is injected to introduce a gene. This results in gene transfer into cells in the left kidney.

Such a method comprises steps of: (a) blocking the blood stream in a renal blood vessel; (b) injecting the Paramyxoviral vector of the present invention into the renal blood vessel; and (c) incubating for infection of the vector. The blood stream to be blocked is, for example, the blood stream in the renal artery. It is preferable to inject the vector while the artery is blocked, block the vein, then carry out incubation.

Specifically, for example, the blood stream to the kidney can be blocked by clamping the aorta immediately below the superior mesenteric artery and above the inferior mesenteric artery. As described above, only the blood stream to either right or left kidney can be blocked. After perfusion with saline, the vector of the present invention can be injected directly from the renal artery using a needle of appropriate gauge. After infection of the vector by incubation for a short time, the clamp is removed to resume the blood stream.

The vector can be injected by inserting a catheter from the common carotid artery and thoracic aorta toward a proximal portion of the renal artery (Tomita, N. et al., Biochem. Biophys. Res. Commun. 186: 129-134, 1992). In this case, for example, the blood stream is blocked at a distal portion of the renal artery by clamping the abdominal aorta. After perfusion with saline, a vector solution is injected *via* the catheter. The vector may be injected via the blood vessel by any other known method.

When introducing the vector from the urinary tract to cause backflow towards a kidney, a syringe or catheter is inserted into the urinary tract to inject a vector solution therefrom. Blood vessels can be blocked with clamps or such during injection. This method comprises steps of: (a) blocking the blood stream of a renal blood vessel; (b) introducing the Paramyxoviral vector of the present invention into the urinary tract; and (c) incubating for infection of the vector. For example, the vector can be injected after the left renal vein is blocked by clamping. Once a catheter is inserted into the urinary tract, a vector solution can be injected near or into the renal pelvis or other region.

Incubation may be carried out, for example, for several minutes (about 1 minute to about 10 minutes), to achieve vector infection. If the blood stream is blocked, it is preferable to resume it in short time that ensures sufficient infectivity. Because such blocking for longer than 10 minutes may produces ischemic injury of tissues. When the blood stream blocking is prolonged, it is preferable to cool the tissues with ice or such to reduce the risk of tissue damages.

Further, the combination of the viral vector with a biocompatible polyol (for example, poloxamer 407 or such) allows to increase the transfer efficiency of the viral vector at 10 to 100 times (March et al., Human Gene Therapy 6: 41-53, 1995). This allows to reduce the dose of the viral vector and the time for infection. In the present invention, the Paramyxoviral vector of the present invention can be provided as a composition by combining with a biocompatible polyol. Another biocompatible polyol can be used in combination. Further, various drugs increasing or decreasing the blood pressure can be administered in combination with the vector. These drugs can be administered together with the vector at the same time, or separately.

Furthermore, the *ex-vivo* gene transfer into renal cells can be achieved according to the method of the present invention (Kitamura, M. et al., J. Clin. Invest. 94: 497-505; Kitamura, M. et al., Kidney Int. 51: 1274-1279, 1997; Naito, T. et al., Mol. Med. 2: 297-312, 1996; Koseki, C. et al., Am. J. Physiol. 261: C550-C554, 1994; Heikkila, P. et al., Gene Ther. 3: 21-27, 1996; Zeigler, S. et al., Transplantation 61: 812-817, 1996). For example, for transplantation, a kidney is surgically removed from a donor, and the vector of the present invention is introduced into this kidney. Alternatively, renal cells are collected from the kidney and the gene is introduced into the cells, followed by injection of the cells into patient's body. Such cells include, for example, mesangial cells, macrophages, and renal tubular epithelial cells. Specifically, for example, mesangial cells may be collected from a kidney of a patient by biopsy, and returned to the patient's kidney after gene transfer. The vector of the present invention can be used to achieve gene transfer into such mesangial cells. Alternatively, *ex-vivo* transfection to the interstitium can be achieved by introducing a gene into renal tubular epithelial cells and carrying out intracapsular transplantation of the cells.

There is no limitation on the type of renal cell that is a target in gene transfer using the Paramyxoviral vector of the present invention. Such renal cells include, for example, cells of the interstitium, glomerulus, blood vessel (including artery, vein and capillary vessel) and renal tubule (including distal renal tubule and proximal renal tubule). Specifically, preferred target cells are interstitial fibroblast cells, mesangial cells, vascular endothelial cells, renal tubular epithelial cells (TEC), macrophages, etc. Such target cells also include adult and fetal renal cells (including cells of metanephros, mesonephros and pronephros). It is also possible to introduce a gene into renal primordial cells such as mesenchymal cells.

There is no limitation on the type of gene that is to be transferred using the Paramyxovirus of the present invention. Such genes may encode naturally occurring proteins or artificial proteins. Naturally occurring proteins include, for example, hormones, cytokines, growth factors, receptors, enzymes, and peptides. Such a protein can be a secretory protein, a transmembrane protein, a cytoplasmic protein, a nuclear protein, etc. Artificial proteins include, for example, fusion proteins such as chimeric toxin, dominant negative proteins (including soluble molecules of receptors and membrane-bound dominant negative receptors), deficient-type cell-adhesion molecules, and cell-surface molecules. Such a protein can comprise an addition of a secretory signal, a membrane localization signal, or a nuclear translocation signal. A gene to be introduced may be a gene which is not originally expressed in renal cells. Alternatively, a gene which is normally expressed in renal cells can be introduced to be overexpressed. It is also possible to suppress the functions of an undesirable gene expressed in renal cells by introducing an antisense RNA molecule or ribozyme that cleaves RNA.

The vector of the present invention can be applied to gene therapy for various renal diseases. Such gene therapy can be carried out, for example, to compensate for the expression defects in cells caused by gene deficiency, to confer a new function by introducing an exogenous gene into cells, or to suppress an undesirable activity in cells by introducing a gene that suppresses the activity of a certain gene. The vector of the present invention is also useful to transfer a gene into nonhuman mammalian renal cells. For example, the vector is useful to establish various disease models and to develop or evaluate therapeutic methods in disease model, in renal transplantation, etc.

The present invention is applicable, for example, to renal transplantation. An exogenous gene can be introduced into a transplant using the vector of the present invention in allotransplantation or xenotransplantation of kidney. For example, it is possible to suppress post-transplantation acute rejection by expressing a gene capable of suppressing the rejection (see, for example, Zeigler, S. et al., Transplantation 61: 812-817, 1996). Alternatively, renal cells such as macrophages, mesangial cells or endothelial cells are isolated as describe above and an exogenous gene is introduced into the cells, which can be transferred into a kidney. The administration of mesangial cells by transplantation can be carried out, for example, by the known mesangial cell-vector system (Kitamura, M. et al., J. Clin. Invest. 94: 497-505, 1994; Kitamura, M. Exp. Nephrol. 5: 118-125, 1997). With this method, the mesangial cells will be trapped in glomerular capillaries and express the exogenous gene. Alternatively, it is possible to use macrophage vectors or such (Kitamura, M. et al., Kidney Int. 51: 1274-1279). For example, genes encoding various cytokines, immunosuppressor proteins or antifibrinolytic proteins may be introduced into inflamed glomerulus.

The present invention can be used to treat kidney cancers. A gene for treating cancers can be introduced into cancerous lesions via an arterial catheter or such. Genes to be used for this purpose include those encoding cytokines and those encoding proteins inhibiting angiogenesis such as soluble vascular endothelial growth factor (VEGF) receptor.

The vector of the present invention can also be used to introduce the gene encoding nitric oxide synthase (NOS) or a gene inhibiting the function of intracellular adhesion molecule-1 (ICAM-1), for treating acute tubular necrosis. It is also possible to use the vector of the present invention for other purposes such as prevention of renal arterial stenosis after angioplasty or treatment of polycystic kidney disease.

Other diseases to which the present invention is applicable include acute glomerulonephritis (AGN) caused by bacterial infection in the upper respiratory tract or such, rapidly progressive glomerulonephritis (RPGN), chronic glomerulonephritis (CGN) (including minimal change, focal glomerular sclerosis, membranous nephropathy, mesangial proliferative glomerulonephritis, membranoproliferative glomerulonephritis and sclerosing glomerulonephritis), secondary glomerular diseases in which the glomerulus is affected secondarily following diabetes, hepatitis, and others, and nephrotic syndrome. The present invention can also be used to treat endocapillary proliferative nephropathy, crescentic nephropathy, and others. It will be also possible to use the present invention to prevent and treat renal disorders caused by infection by human immunodeficiency virus (HIV) or such.

Further, specific examples of diseases or disorders which can be treated by the vector of the present invention targeting renal cells, and candidate genes for the treatment are as follows:

| | |
|---|---|
| 1. chronic glomerulonephritis | hepatocyte growth factor (HGF), Smad 6/7, dominant negative TGF-β receptor |
| 2. diabetic nephropathy | hepatocyte growth factor (HGF), Smad 6/7, dominant negative TGF-β receptor |
| 3. glomerulosclerosis | hepatocyte growth factor (HGF), Smad 6/7, dominant negative TGF-β receptor |
| 4. Alport syndrome | type IV collagen |
| 5. tubulointerstitial nephritis | hepatocyte growth factor (HGF), Smad 6/7, dominant negative TGF-β receptor |

Transforming growth factor β (TGF-β) is a multi-functional cytokine, and plays important roles in tissue turnover, restoration of damaged tissues, and so on. In particular, TGF-β induces the accumulation of extracellular matrix (ECM) as well as regulates the expression of ECM receptors such as integrin. An event shared by various progressive renal diseases including diabetic nephropathy is the accumulation of extracellular matrix (ECM) in the glomerular. Thus, the accumulation of ECM can be suppressed by inhibiting the expression or function of TGF-β.

For example, decorin, which is one of small molecule dermatan sulfate proteoglycans, is a natural inhibitory factor to TGF-β. The introduction of the gene (decorin cDNA) results in the reduction of the accumulation of ECM in the glomerular (Isaka, Y. et al., Nat. Med. 2: 418-423, 1996).

Furthermore, a molecule comprising the extracellular domain of TGF-β receptor neutralizes TGF-β and inhibits the function of TGF-β. There are two types of TGF-β receptors, type I and type II. TGF-β binds to the type II receptor, and the resulting complex interacts with the type I receptor. Both type I and type II receptor fragments can be used to suppress TGF-β, but the type II receptor fragment that directly interacts with TGF-β is used preferably. TGF-β can be neutralized by expressing an inactivated TGF-β receptor in which the signaling function has been disrupted. A certain effect can be produced by simply expressing the soluble receptor (Lin, H. Y. et al., J. Biol. Chem. 270: 2747-2754, 1995). However, significantly stronger effects can be produced by expressing, for example, a fusion molecule of the extracellular domain of TGF-β receptor with IgG-Fc (for example, TGF-RII/Fc)(Isaka, Y. et al., Kidney Int. 55: 465-475, 1999; Isaka, Y. et al., J. Am. Soc. Nephrol. 7: 1735, 1996). The accumulation of ECM in the glomerular can be suppressed by expressing this molecule in renal cells. Likewise, it is possible to use a fusion protein of the extracellular domain of platelet-derived growth factor (PDGF) with Fc (Imai, R. and Y. Isaka, Nephron 83: 296-300, 1999; Isaka et al., Kidney Int. 52: Suppl. S100-S103, 1997). An antisense RNA molecule or ribozyme cleaving RNA can be introduced to suppress the expression of these genes. In contrast, the introduction of the TGF-β gene may produce a therapeutic effect on acute glomerular damages or such. The TGF-β gene can also be used to establish a renal disease model.

Smad is a family of signaling molecules which transduce the TGF-β signal. The function of this gene family is regulated by gene transfer. For the suppression of the action of TGF-β, it is particularly preferable to introduce the suppressive Smad gene, which suppressively regulates the TGF-β signal. Such molecules include Smad6 and Smad7.

Hepatocyte growth factor (HGF) is a growth factor produced in mesenchymal cells, and regulates the proliferation of endothelial cells and epithelial cells in organs including liver and kidney. HGF also induces morphogenesis to form branched renal tubules. Additionally, HGF prevents acute kidney failure caused by ischemic or drug-induced tissue injury (Kawaida, K. et al., Proc. Natl. Acad. Sci. USA 91: 4357-61, 1994). HGF therapy using mouse model for renal disease has been performed (Mizuno, S. et al., J. Clin. Invest. 101: 1827-34, 1998). Thus, HGF gene is important as a transgene in gene therapy targeting renal cells (Imai, R. and Y. Isaka, Nephron 83: 296-300, 1999). HGF is also useful to prevent progressive fibrosis associated with renal diseases.

15-lipoxygenase (15-LO) is an enzyme that catalyzes the synthesis of 15-S-hydroxyeicosatetraenoic acid and lipoxin A₄ which antagonize the inflammatory action of leukotriene. The introduction of the 15-LO gene reportedly produces therapeutic effects in a glomerulonephrosis model (Yura, T. et al., J. Am. Soc. Nephrol. 6: 890, 1995; Imai, E. et al., Exp. Nephrol. 5: 112-117, 1995; Munger, K. A. et al., Proc. Natl. Acad. Sci. USA 96: 13375-13380, 1999); thus, this gene can also be used for therapeutic purposes.

Exogenous genes that are particularly useful in gene transfer targeting renal cells (e.g. in gene therapies) may include those encoding:
(1) cell cycle inhibitor (e.g. p53, p21, p16 and p27),
(2) inhibitor of cell proliferation signal (e.g. mutant H-Ras),
(3) secretory cell proliferation inhibitor (e.g. eNOS and CNP (C type sodium diuretic peptide)),
(4) vascular smooth muscle relaxing factor (e.g. eNOS and CNP),
(5) vascular smooth muscle relaxing ion channel (e.g. C-terminal deletion mutant of Kir 6.2 potassium ion channel),
(6) thrombolytic protein (e.g. tissue plasminogen activator and urokinase),
(7) tissue factor pathway inhibitor (TFPI),
(8) angiogenic factor (e.g. VEGF, FGF, and HGF), and
(9) superoxide dismutase (SOD) (e.g. including Cu/Zn type SOD, Mn type SOD, and EC-SOD)

One of advantages of the vector of the present invention is to achieve highly efficient gene transfer into renal interstitial cells. This advantage would enable gene therapy for various renal diseases. All human progressive renal diseases finally result in tubulointerstitial fibrosis. Persistent inflammation or recurrent injuries of the interstitium gradually damage kidney tissues, which often results in severe renal malfunction (Schainuck, L. I. et al., Hum. Pathol. 1: 631-641, 1970; Bohle, A. et al., Virchows Arch. 376: 211-232; Bohle, A. et al., Virchows Arch. 373: 15-22, 1977; Mackensen, S. et al., Nephron 24: 30-34, 1979). Interstitial fibrosis can sometimes be caused secondarily by glomerular or vascular damages. The detailed mechanism underlying the progress of a renal disease to the final stage of renal failure has not been clarified. Some studies reported that the degree of not glomerular damage but tubulointerstitial damage can exactly correlates to the degrees of renal dysfunction and long-term prognosis (Risdon, R. A. et al., Lancet 2: 363-366, 1968; Schainuck, L. et al., Hum. Pathol. 1: 631-641, 1970).

The process leading to interstitial fibrosis can be assumed to be attributed to an aberrant prolongation of the normally regulated metabolic turnover of cell components and extracellular matrix. The interstitial fibroblast acquires the compound phenotype of so-called "myofibroblast" and, proliferatesto infiltrate in residential areas which are actively inflamed (Alpers, C. et al., J. Am. Soc. Nephrol. 5: 201-210, 1994). Then, these cells induce the synthesis of extracellular matrix and enhance local fibrosis. Accordingly, the gene transfer technology targeting interstitial fibroblast allows evaluation ofe *in-vivo* effects of a particular molecule in the interstitium. Furthermore, this technology is also important to develop a method of treating interstitial fibrosis.

Gene therapy can be performed by *in vivo* administration of a composition comprising the Paramyxovirus vector directly or indirectly into a kidney to express an exogenous gene in renal cells. Alternatively, the therapy may be carried out by *ex-vivo* administration. The *in-vivo* gene transfer can be achieved, for example, by local administration, such as injection of the vector from the renal artery or renal vein, direct injection into a kidney, backflow from the urinary tract, and backflow from the renal pelvis. In addition, indirect administration into renal cells can be achieved, for example, by systemic administration or administration to other organs. The *ex-vivo* gene transfer can be achieved, for example, by administration of the vector to the surgically removed kidney, in such a manner as injection into the renal artery or renal vein, direct injection into the kidney, backflow from the urinary tract, and backflow from the renal pelvis. Alternatively, the gene can be introduced by preparing renal cells from the organ or such, and incubating the cells with the vector of the present invention. The renal cells into which the gene has been introduced can be returned to, for example, the kidney.

For treatment and prevention, the Paramyxoviral vector for gene transfer into renal cells is administered at a sufficient dose that ensures the introduction of an effective amount of the vector into renal cells. The term "effective amount" refers to an amount that ensures the introduction of the gene into renal cells by the method of the present invention, which produces a desired therapeutic or preventive effect at least in part. The administration of an effective amount of the Paramyxoviral vector of the present invention comprising a desired gene results in the production of the transgene product in cells where the vector has been introduced. This induces alterations in the phenotypes of the cells and/or the surrounding renal cells (specifically, induction or suppression of the expression of the transgene and other genes, and the morphological, biochemical alterations, etc.). Preferably, the administration of an effective amount of the vector of the present invention comprising the desired gene to a kidney leads to the introduction of the gene into a significant number of cells in the kidney as well as the induction of alterations in the phenotypes of the cells. The term "a significant number of cells" means that the gene has been introduced by the vector of the present invention into at least about 0.1%, preferably about 1% or more, more preferably about 5% or more, still more preferably about 10% or more, most preferably about 20% or more of target renal cells at the administration site. The target renal cells include, for example, interstitial cells, glomerular cells, renal tubular cells, mesangial cells, epithelial cells, and endothelial cells.

The achievement of gene transfer into cells can be confirmed by an assay method known to those skilled in the art. The transcript of a gene can be detected and quantified, for example, by Northern hybridization, reverse transcription-polymerase chain reaction (RT-PCR), or RNA protection assay. The detection by Northern hybridization, RT-PCR or such can also be carried out *in situ.* The detection of the translation product can be carried out by Western blotting using an antibody, immunoprecipitation, RIA, enzyme-linked immunosorbent assay (ELISA), pull-down assay, etc. To easily detect the achievement of gene transfer, the protein to be expressed can be tagged or a reporter gene can be inserted so as to ensure its expression. The reporter gene includes, but not limited to, genes encoding β-galactosidase, chloramphenicol acetyltransferase (CAT), alkaline phosphatase, and green fluorescent protein (GFP).

A dose of the vector may vary depending on the disease, the body weight, age, sex, symptom, the purpose of administration, the transgene, a formulation of a composition to be administered, a method of administration, and so on, but it can be appropriately determined by those skilled in the art. The dose of the vector may be preferably within the range of approximately 10⁵ pfu/ml to 10¹¹ pfu/ml, and more preferably approximately 10⁷ pfu/ml to 10⁹ pfu/ml, but most preferably approximately 1 x 10⁸ pfu/ml to 5 x 10⁸ pfu/ml, with pharmaceutically acceptable carriers.

The composition of the present invention comprising the virus vector may be administered into subjects including all mammalian animals including humans, monkeys, mice, rats, rabbits, sheep, cattle, and dogs.

### Brief Description of the Drawings

Fig. 1 is a photograph showing the result observed under a fluorescence stereoscopic microscope, which shows a frontal section of the rat kidney surgically removed on the fourth day after administration of SeV/GFP from the renal artery. Intense fluorescent signals are detected in a wide area of cortical part as well as medullar part.

Fig. 2 is a photograph showing the result observed under a fluorescence stereoscopic microscope, which shows a frontal section of the rat kidney surgically removed on the fourth day after administration of SeV/GFP from the urinary tract. Intense fluorescent signals are detected in a wide area of cortical part as well as medullar part.

Fig. 3 is photographs of a kidney tissue section stained with an antibody against GFP. The section was obtained on the fourth day after administration of SeV/GFP from the renal artery. Positive signals are diffusively detected in interstitial fibroblast cells. Each part of the tissue is assigned in the lower panel.

Fig. 4 presents photographs of a kidney tissue section stained with an antibody against GFP. The section was obtained on the fourth day after administration of SeV/GFP from the urinary tract. Positive signals are diffusively detected in interstitial fibroblast cells. Each part of the tissue is assigned in the lower panel.

Fig. 5 presents photographs of a kidney tissue section stained with an antibody against GFP. The section was obtained on the fourth day after administration of SeV/GFP from the renal artery. GFP antibody-positive stains were also recognized in the glomerulus; about 20% of the glomerulus was found to be infected with the vector. Glomerular portions are indicated in the lower panel. Infiltrating cells are shaded.

Fig. 6 presents photographs of a kidney tissue section stained with an antibody against GFP. The section was obtained on the fourth day after administration of SeV/GFP from the urinary tract. Positive signals are detected in renal tubular epithelial cells. In addition, macrophages are found to infiltrate. Each part of the tissue and cell are assigned in the lower panel. Infiltrating cells are shaded.

Fig. 7 is a photograph of a kidney tissue section stained with an antibody against GFP. The section was obtained on the seventh day after administration of SeV/GFP from the renal artery. GFP antibody-positive stains are recognized in glomeruli.

Fig. 8 is a photograph of a kidney tissue section stained with an antibody against GFP. The section was obtained on the seventh day after administration of SeV/GFP from the renal artery. GFP antibody-positive stains are recognized in interstitial cells and glomeruli.

Fig. 9 is a photograph of a kidney tissue section stained with an antibody against GFP. The section was obtained on the seventh day after administration of SeV/GFP from the renal artery. GFP antibody-positive stains are recognized.

Fig. 10 is a photograph of a kidney tissue section stained with an antibody against GFP. The section was obtained on the 14th day after administration of SeV/GFP from the renal artery.

Fig. 11 is a photograph of a kidney tissue section stained with an antibody against GFP. The section was obtained on the 14th day after administration of SeV/GFP from the renal artery.

Fig. 12 is a photograph of a kidney tissue section stained with an antibody against GFP. The section was obtained on the 14th day after administration of SeV/GFP from the renal artery.

Fig. 13 is a photograph of a kidney tissue section stained with an antibody against GFP. The section was obtained on the 14th day after administration of SeV/GFP from the renal artery.

Fig. 14 is a photograph of a kidney tissue section stained with an antibody against GFP. The section was obtained on the 28th day after administration of SeV/GFP from the renal artery.

Fig. 15 is a photograph of a kidney tissue section stained with an antibody against GFP. The section was obtained on the 28th day after administration of SeV/GFP from the renal artery.

### Best Mode for Carrying out the Invention

The present invention will be explained in detail below with reference to Examples, but it is not to be construed as being limited thereto. All references and publications (including references, patents and published patent applications) cited herein are incorporated herein by reference in their entireties.

### [Example 1] Construction and preparation of recombinant Sendai virus vector comprising the GFP gene (GFP/SeV) for kidney administration

The recombinant Sendai virus vector comprising the GFP gene (GFP/SeV) was constructed based on the description of Kato, A. et al., 1997, EMBO J. 16: 578-587 and Yu, D. et al., 1997, Genes Cells 2: 457-466, as follows:

First, a DNA sample comprising the cDNA nucleotide sequence of the GFP gene (the length of structure gene = 717 bp), was prepared. The DNA sample was purified until the plasmid was observed as a single band at 25 ng/µl or a higher concentration in electrophoresis. In order to amplify and collect a desired gene fragment from the sample, a primer pair comprising synthetic forward DNA sequence and reverse DNA sequence (antisense strand) were prepared; the primers contained a NotI restriction site sequence; the below-described transcription end sequence (E), intervening sequence (I), and transcription start sequence (S); and a portion of the GFP gene sequence.

The forward synthetic DNA sequence contains ACTT at the 5'-terminus, the NotI recognition site "GCGGCCGC" at the 3'-side thereof, and a spacer sequence at the 3'-side thereof. Further to the 3'-side thereof, the sequence corresponding to the first 25 nucleotides of the ORF starting from the initiation codon ATG of a desired cDNA was attached.

The reverse synthetic DNA sequence contains ACTT at the 5'-terminus the NotI recognition sequence "GCGGCCGC" at the 3'-side thereof, and a spacer oligo DNA for adjusting the length of the primer at the 3'-side thereof. The length of the oligo DNA was designed so as to be a multiple of 6 nucleotides including the NotI recognition sequence GCGGCCGC, the sequence complementary to the cDNA, and the EIS sequence derived from the Sendai virus genome as described below (so-called "rule of six"; Kolakofski D. et al., J. Virol., 1998, 72, 891-899; Calain P. and Roux L., J. Virol., 1993, 67, 4822-4830). Furthermore, to the 3'-side of the added sequence, complementary sequences to the S sequence of the Sendai virus (5'-CTTTCACCCT-3' (SEQ ID NO: 1)), to the I sequence (5'-AAG-3') and to the E sequence (5'-TTTTTCTTACTACGG-3' (SEQ ID NO: 2)) were added. Finally, to the 3'-side, the complementary sequence corresponding to 25 nucleotides upstream from the termination codon of the desired cDNA was added to be a 3'-teminus of the reverse synthetic oligo DNA.

PCR was performed with Vent polymerase (NEB). The amplified fragment was digested with NotI, and inserted into the NotI site of the plasmid vector pBluescript. The nucleotide sequence of the obtained PCR product was confirmed by an automated DNA sequencer, and a plasmid having the correct sequence was selected.

Recombinant Sendai virus genomic cDNA was constructed according to the methods described in Kato A. et al., EMBO J., 1997, 16, 578-598 and Hasan M. K. et al., J. Gen. Virol., 1997, 78, 2813-2820. A spacer sequence of 18 bp containing the NotI site (5'-(G)-CGGCCGCAGATCTTCACG-3'; SEQ ID NO: 3) was inserted into a cloned Sendai virus genomic cDNA (pSeV(+)) at the locus between the leader sequence and the 5'-terminus of a sequence encoding the N protein to obtain the plasmid pSeV18⁺b(+) containing a self-cleavable ribozyme site derived from the antigenomic strand of the hepatitis delta virus (Hasan M. K. et al., J. General Virol., 1997, 78, 2813-2820). The above-mentioned DNA encoding GFP was digested with NotI, and inserted into the cDNA plasmid of Sendai virus genome pSeV18⁺b(+) at the NotI site to obtain a recombinant Sendai virus cDNA comprising the GFP gene.

The recombinant Sendai virus cDNA prepared as described above was transcribed *in vitro* or in cells to reconstitute the virus, thereby obtaining the recombinant viral vector as follows.

Cells of the monkey kidney cell line LLCMK2 were cultured to be 70 to 80% confluent (1x10⁶ cells) in minimal essential medium (MEM) comprising 10% fetal calf serum (FCS) and antibiotics (100 units/ml penicillin G and 100 µg/ml streptomycin) in 6-well plastic plates. The recombinant vaccinia virus vTF7-3 expressing T7 polymerase was inactivated by UV irradiation (Fuerst, T. R. et al., Proc. Natl. Acad. Sci. USA 83: 8122-8126, 1986, Kato, A. et al., Genes Cells 1: 569-579, 1996), and the cells were infected with the virus at 2 PFU/cell. One hour after infection, 3 to 5 µg recombinant Sendai virus cDNA was co-transfected with plasmids (1 µg pGEM-N, 0.5 µg pGEM-P, and 1 µg pGEM-L)(Kato, A. et al., Genes Cells 1: 569-579, 1996) expressing trans-acting viral proteins essential for the production of full-length Sendai virus genome by lipofection using Superfect (QIAGEN). The transfected cells were cultured in serum-free MEM supplemented with only 100 µg/ml rifampicin (Sigma) and 40 µg/ml cytosine arabinoside (AraC) (Sigma). Optimal concentrations for these agents were determined so as to minimize the cytotoxicity of vaccinia virus and to maximize virus yield (Kato, A. et al., 1996, Genes Cells 1: 569-579). 48 hours after transfection, the cells were harvested and disrupted by three cycles of freeze-thawing, and then inoculated into the allantoic membrane of 10-day-old embryonated eggs. After three days, the allantoic fluid was collected, and the virus titer was determined by measuring hemagglutination activity (HA). HA was determined by the "endo-point dilution method" (Kato, A. et al., 1996, Genes Cells 1: 569-579; Yonemitsu, Y. & Kaneda, Y., "Hemagglutinating virus of Japan-liposome-mediated gene delivery to vascular cells.", Baker A. H. (ed.), Molecular Biology of Vascular Diseases. Method in Molecular Medicine, Humana Press, pp. 295-306, 1999). In order to exclude vaccinia virus vTF7-3 samples whose titers might be 10³ to 10⁴ pfu/ml or lower, the allantoic fluid samples were diluted 10⁶ fold, and the dilutes were used to re-propagate the viruses in embryonated eggs. This procedure was repeated three times or more. The allantoic fluids obtained were stored at 4°C as sample solutions for administration to kidney. Based on the HA value, the virus titer was estimated to be 2x 10⁹ pfu/ml.

### [Example 2] Construction and preparation of Sendai virus vector lacking the F gene

### (1) Construction of Sendai virus genome cDNA lacking the F gene and an expression plasmid for F gene

Full length Sendai virus (SeV) genomic cDNA, pSeV18⁺b(+) (Hasan M. K. et al., J. General Virol., 1997, 78, 2813-2820) (pSeV18⁺b(+) may be also called pSeV18⁺), was digested with SphI and KpnI, and the resulting fragment (14673 bp) was recovered and cloned into pUC18 to obtain pUC18/KS. pUC18/KS was used for constructing a region lacking the F gene. Deletion of the F gene was performed by combination of PCR and ligation, and the ORF of the F gene (1698 bp, from ATG to TGA) was replaced with the sequence 5'-atgcatgccggcagatga (SEQ ID NO: 4) to construct the F gene-deleted SeV genomic cDNA (pSeV18⁺/ΔF). PCR was performed using the forward primer (5'-gttgagtactgcaagagc/SEQ ID NO: 5) and the reverse primer (5'-tttgccggcatgcatgtttcccaaggggagagttttgcaacc/SEQ ID NO: 6) for amplification of the upstream of the F gene, and the forward primer (5'-atgcatgccggcagatga/SEQ ID NO: 7) and the reverse primer (5'-tgggtgaatgagagaatcagc/SEQ ID NO: 8) for amplification of the downstream of the F gene, and the PCR products were ligated using EcoT22I. The resulting plasmid was digested with SacI and SalI, and the fragment containing the F gene deletion site (4931 bp) was recovered and cloned into pUC18 to obtain pUC18/dFSS. pUC18/dFSS was digested with DraIII, and the fragment recovered was replaced with the DraIII fragment of pSeV18⁺ comprising F gene, and ligated to obtain pSeV18⁺/ΔF.

Then, the EGFP gene was amplified by PCR to construct a cDNA comprising the EGFP gene at the F deletion site (pSeV18⁺/ΔF-GFP). The length of EGFP gene was adjusted to be a multiple of 6 (Hausmann, S. et al., RNA 2, 1033-1045 (1996)) by PCR using a NsiI-tailed primer (5'-atgcatatggtgatgcggttttggcagtac:sequence SEQ ID NO: 9) for 5'-terminus, and a NgoMIV-tailed primer (5'-tgccggctattattacttgtacagctcgtc:sequence SEQ ID NO: 10) for 3'-terminus. The PCR products were digested with restriction enzymes NsiI and NgoMIV. The resulting fragments were recovered from the gel and then ligated with pUC18/dFSS between the restriction enzyme sites of NsiI and NgoMIV located at the F deletion site, followed by sequencing. A DraIII fragment comprising the EGFP gene was excised from the plasmid and was substituted for the DraIII fragment located in a region comprising the F gene in pSeV18⁺. After ligation, the plasmid pSeV18⁺/ΔF-GFP was obtained.

### (2) Preparation of helper cells inducibly expressing the SeV-F protein

A Cre/loxP inducible expression plasmid for the F gene of Sendai virus (SeV-F) was constructed as follows. The SeV-F gene was amplified by PCR and its sequence was confirmed. The PCR product was inserted into the unique SwaI site of the pCALNdlw plasmid, which had been designed so that the expression of gene products could be induced by Cre DNA recombinase (Arai, T. et al., J. Virol. 72, 1115-1121 (1998)). The plasmid pCALNdLw/F was thus obtained.

To recover infective virus particles from the F gene-deleted genome, a helper cell line expressing SeV-F protein was established. LLC-MK2 cells, derived from the Simian kidney and commonly used for SeV propagation, were used. LLC-MK2 cells were cultured at 37°C under 5% CO₂ in MEM containing 10% heat-inactivated and immobilized fetal bovine serum (FBS), 50 U/ml of penicillin G sodium, and 50 µg/ml streptomycin. Because of the cytotoxicity of the SeV-F gene product, the gene was cloned into the pCALNdLw, where the expression of a cloned gene was inducible by Cre DNA recombinase. The above pCALNdLw/F was used to transfect LLC-MK2 cells by the calcium phosphate method (mammalian transfection kit (Stratagene)) according to the protocol.

LLC-MK2 cells grown in 10-cm plates to be 40% confluent were transfected with 10 µg pCALNdLw/F and incubated in 10 ml of MEM containing 10 % FBS at 37°C under 5% CO₂ for 24 hr. Then, cells were detached, resuspended in 10 ml of culture medium, and plated onto five 10-cm dishesso that 5 ml of cell suspension was plated onto one dish, 2 ml onto two, and 0.2 ml onto two. Cells were cultured in 10 ml of MEM containing 10% FBS plus 1200 µg/ml G418 (GIBCO-BRL) for 14 days while the medium was changed every two days, and stable transfectants were selected. Thirty clones grown in the medium that are resistant to G418 were recovered using cloning rings. Cells of each clone were further cultured to be 100% confluent in a 10-cm dish.

To induce F protein expression, cells were grown to be 100% confluent in 6 cm dishes, and infected with AxCANCre adenovirus at moi = 3 according to the method by Saito et al. (Saito et al., Nucleic Acids Res., 1995, 23, 3816-3821; Arai T. et al., J. Virol., 1998, 72, 1115-1121).

### (3) Reconstitution and propagation of the F gene-deleted SeV virus

LLC-MK2 cells were transfected with the plasmid pSeV18⁺/ΔF-GFP described above, in which the enhanced green fluorescent protein (EGFP) gene as a reporter had been inserted into the F deletion site according to the 6n rule, as follows.

LLC-MK2 cells were plated at 5x 10⁶ cells/dish onto 100-mm petri dishes, cultured for 24 hr, and then infected at room temperature for 1 hr with the recombinant vaccinia virus expressing T7 RNA polymerase, which had been treated with psoralen and long UV (365 nm) for 20 min (Fuerst T. R. et al., Proc. Natl. Acad. Sci. USA, 1986, 83, 8122-8126) (moi = 2) (preferably moi = 2 to 3; more preferably moi = 2). UV exposure was performed using UV Stratakinker 2400 equipped with five 15-watt bulbs (catalogue number 400676 (100 V), Stratagene, La Jolla, CA, USA). After cells were washed three times, plasmids pSeV18⁺/ΔF-GFP, pGEM/NP, pGEM/P, and pGEM/L (Kato A. et al., Genes Cells, 1996, 1, 569-579) were resuspended in OptiMEM (GIBCO) at a ratio of 12 µg/dish, 4 µg/dish, 2 µg/dish, and 4 µg/dish, respectively, and mixed with SuperFect transfection reagent (5 µl SuperFect (QIAGEN) for 1 µg DNA). The mixture was incubated for 10 min at room temperature, then resuspended in 3 ml of OptiMEM containing 3% FBS, and added to the cells. After a 3-hr culturing in an incubator, cells were washed twice with serum free MEM, and further cultured in MEM containing 40 µg/ml of cytosine β-D-arabinofuranoside (AraC, Sigma) and 7.5 µg/ml of trypsin (GIBCO) for 70 hr. Then, cells were collected and resuspended in OptiMEM at 10⁷ cells/ml. Cells were frozen-thawed three times, mixed with lipofection reagent DOSPER (Boehringer mannheim) (10⁶ cells per 25 µl DOSPER), incubated at room temperature for 15 min, and used to transfect LLC-MK2/F7 cells (10⁶ cells/well in 12-well-plate), which were one of the clones of F gene-expressing helper cells selected as described above. The cells were cultured in serum free MEM containing 40 µg/ml of AraC and 7.5 µg/ml of trypsin, and the culture supernatant was collected.

### [Example 3] In-vivo administration of Sendai virus vector into kidney

### (1) Administration of the virus from the renal artery

Six-week-old Sprague Dawley (SD) rats (male, weight 160 to 180 g) were used. The animals were anesthetized with Nembutal; the original solution of Nembutal was diluted 10 fold with saline, and then given to the peritoneal cavity in a 1/100 ml volume of the weight. The animals were laparotomized by median incision. The left kidney and the renal artery were exposed by removing adhered tissues such as adipose tissues. A 24-G catheter was inserted into the left renal artery to perfuse with physiological saline and administer the virus. Furthermore, to prevent the backflow to the abdominal aorta, the renal artery was clamped at a portion proximal to the abdominal aorta. Kidney perfusion was carried out using several hundreds µl of physiological saline. After perfusion with saline, 300, 500 or 700 µl of a sample solution comprising the virus (titer 2x 10⁹ pfu/ml), which had been prepared in Example 1, was administered with 1-ml syringes. After the catheter was removed, the puncture was closed using Aronalpha™ (Toagosei Co., Ltd.). After 5 minutes, the clamp was removed and the abdomen was closed using a Michel's clip.

### (2) Administration of the virus from the urinary tract

Six-week old Sprague Dawley (SD) rats (male, weight 160-180 g) were used. The animals were anesthetized with Nembutal; the original solution of Nembutal was diluted 10 fold with saline, and then given to the peritoneal cavity in a 1/100 ml volume of the weight. The animals were laparotomized by median incision. The left kidney and the urinary tract were exposed. A 26-G needle was inserted into the urinary tract, and then 300, 500 or 700 µl of a sample solution comprising the virus (GFP/SeV) (titer 2x 10⁹ pfu/ml), which had been prepared in Example 1, was administered to the kidney, while the left renal vein was clamped. After 5 minutes, the clamp was removed and the abdomen was closed using a Michel's clip.

### (3) Collection of kidney samples and preparation of sections

The kidney samples were collected 4, 7, 14, 21 and 28 days af ter viral administration. The rats were anesthetized with Nembuta 1 and then laparotomized. In order to perfuse the kidney, the upp er part of the abdominal aorta, which branches into the renal arte ry, was blocked with an artery forceps, and a winged needle was in serted into the lower part of the abdominal aorta, which branches into the renal artery. Physiological saline (20 ml) was injected into the artery to remove blood, then the kidney was surgically r emoved. The kidney was sliced into frontal sections, and fixed in a methacarn solution (methanol : chloroform : acetic acid = 9:6:1 ) prepared immediately before resection. The fixed sample was emb edded in paraffin according to the standard method, and 4-µm thick ness of paraffin sections were prepared. Deparaffinization was ca rried out using xylene and a serial dilution of ethanol.

### (4) Immunohistochemical staining

The reactivity to anti-GFP antibody (rabbit polyclonal antibody; Molecular Probes, Oregon, USA) and anti-ED-1 antibody (mouse monoclonal antibody, Serotec, Oxford, England) against macrophage was determined to identify virus-infected tissues and to evaluate infiltration into cells. The sections prepared by the method described above were treated with a mixed solution of methanol and hydrogen peroxide (9:1) at room temperature for 5 minutes to inactivate the endogenous peroxidase. After washing three times with PBS solution (for 10 minutes), the sample sections were treated with ablocking agented for 20 minutes at room temperature (the blocking agent was selected from the following combination: 5% goat serum for the rabbit antibody; 5% horse serum for the mouse monoclonal antibody; 200 µl each). The sections were incubated at 4°C for 16 hours with each of the primary antibodies that had been diluted 50 fold. The section samples were washed three times with cold PBS, and then incubated with a secondary antibody. The secondary antibodies used were: biotinylated goat anti-rabbit IgG(H+L) antibody (Vector Laboratories Inc., Burlingame, CA) for confirming the presence of GFP (diluted 150 times); and biotinylated horse anti-mouse IgG(H+L) antibody (Vector Laboratories Inc., Burlingame, CA) for confirming the presence of macrophage (diluted 150 fold) . The reaction with the antibodies was conducted at room temperature for 30 minutes. The sections were washed three times with a cold PBS solution for 10 minutes. The samples were incubated with avidin-biotinylated (Vector; according to the instructions attached to the avidin-biotinylation kit) horseradish peroxidase complex at room temperature for 30 minutes. Coloration reaction was carried out with a DAB solution. After the termination of the reaction, the sample sections were counterstained with methyl green.

### (5) GFP expression on non-fixed kidney sections four days after virus administration observed under fluorescence microscopy

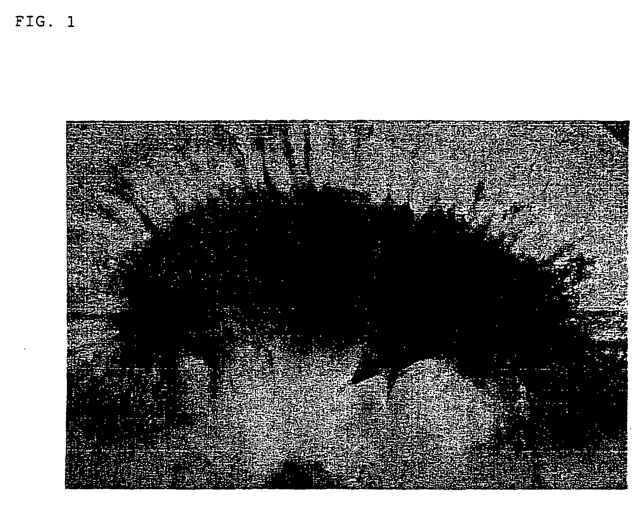

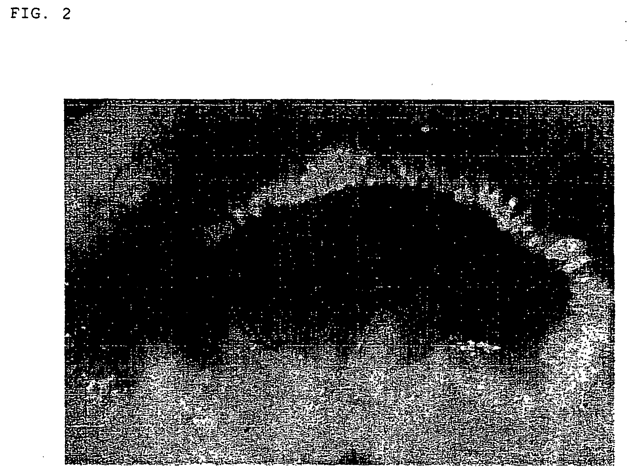

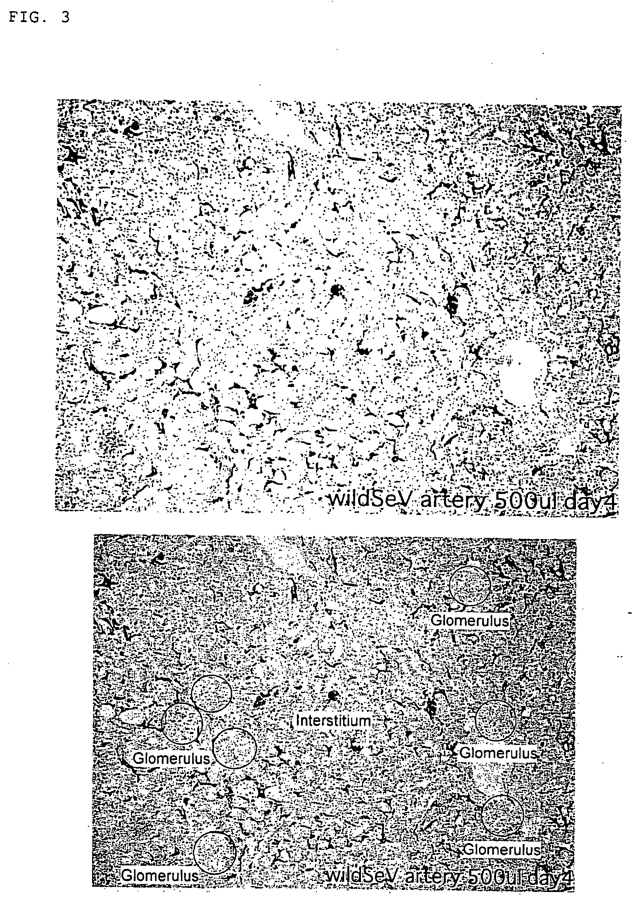

Frontal sections were prepared from the surgically removed non-fixed kidney and observed under a fluorescence stereoscopic microscope (light source for fluorescence: excitation wavelength, 425nm; emission wavelength, 480nm) to fluorescently confirm the expression of GFP derived from the administered GFP/SeV. All the samples used for the fluorescence observation were obtained 4 days after administration of 500 µl of virus liquid from the renal artery or urinary tract. The results are shown in Fig. 1 (administration from the renal artery) and Fig. 2 (administration from the urinary tract). Intense fluorescence signals were detected over a wide area of cortical part as well as medullar part.

### (6) Immunohistochemical staining of samples 4 days after administration

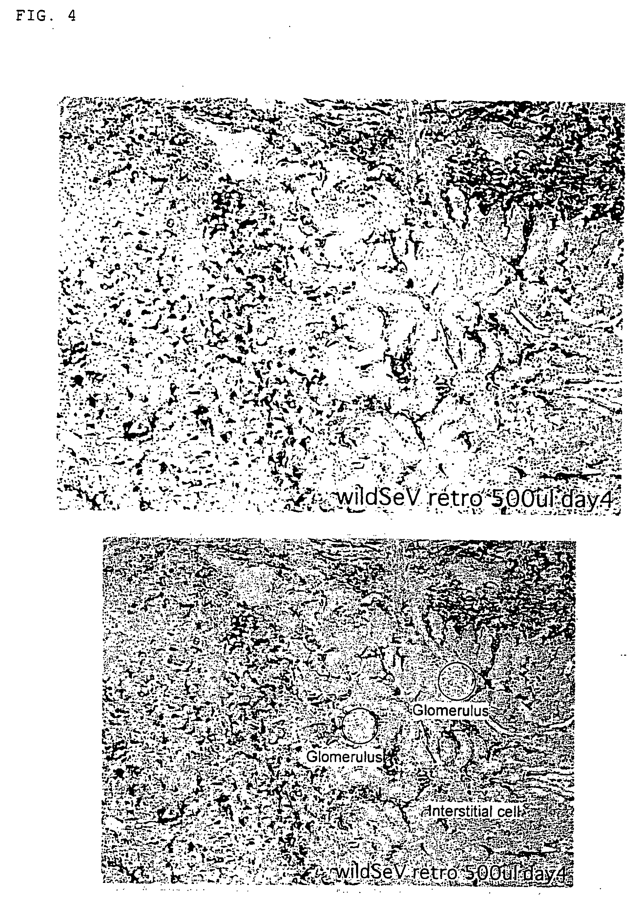

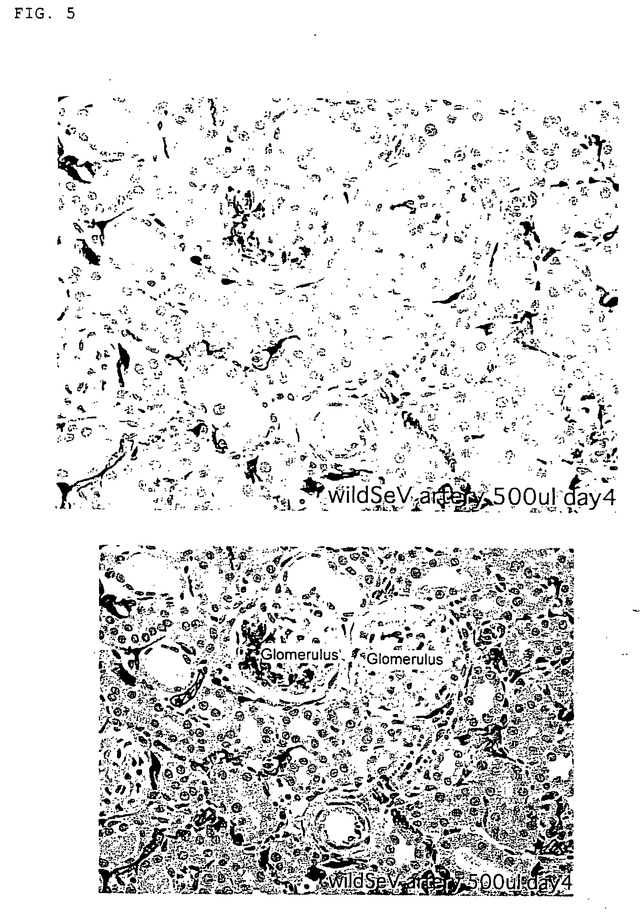

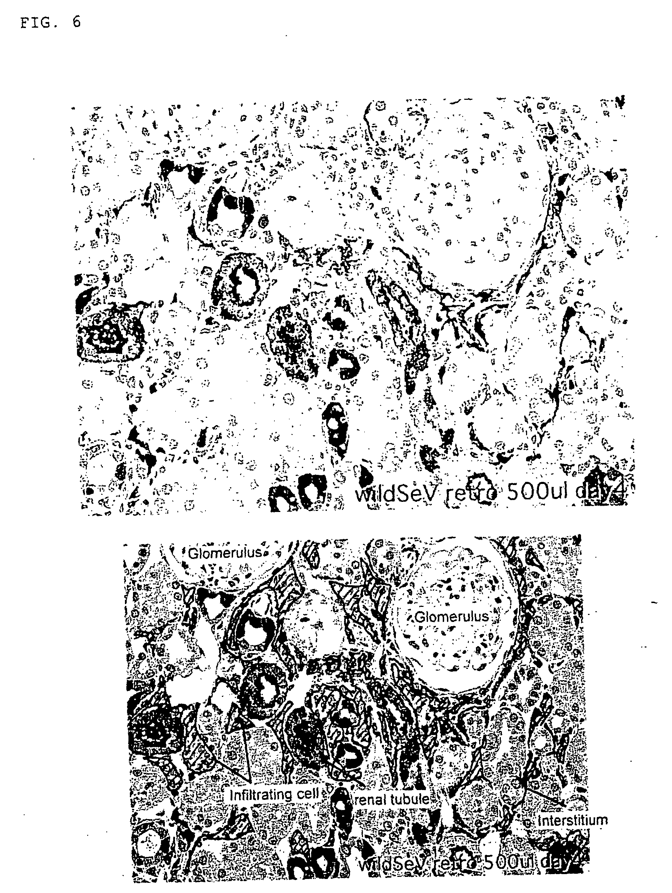

The result obtained using the anti-GFP antibody shows that, when the virus administered from the renal artery or urinary tract, interstitial fibroblast cells were diffusively infected with the virus (Figs. 3 and 4). In the case of administration from the renal artery, GFP antibody-positive stains were recognized in the glomeruli; infected glomeruli were found to be about 20% of the total glomeruli (Fig. 5). The antibody-positive cells in the glomerulus were thought to be mesangial cells. It should be noted that no infiltration of inflammatory macrophages is found in GFP-positive glomeruli and near interstitial cells obtained by administration from the renal artery. In the case of administration from the urinary tract, strong GFP expression was detected in epithelial cells of renal pelvis, and infected cells were observed diffusively among interstitial cells and renal tubular epithelial cells (Fig. 6). In the case of administration from the urinary tract, the expression was found not only in distal renal tubule but also in the proximal renal tubule. Furthermore, in the case of administration from the urinary tract, a considerable number of macrophages infiltrated in tissues surrounding the renal tubule. The difference in the degree of induction of infiltration between the administration routes can be attributed in part to the activity of renal tubular epithelial cells as the antigen-presenting cells.

### (7) Immunohistochemical staining of samples 7, 14, 21 and 28 days after administration

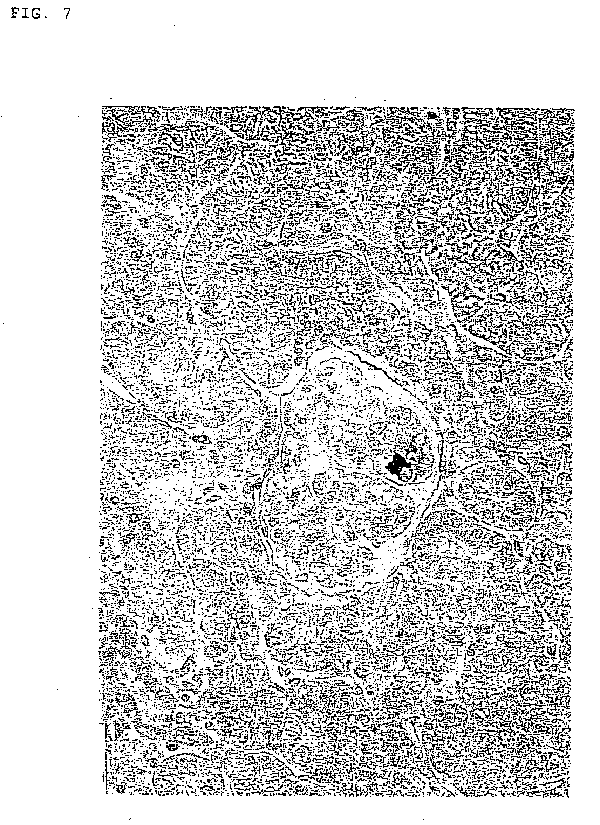

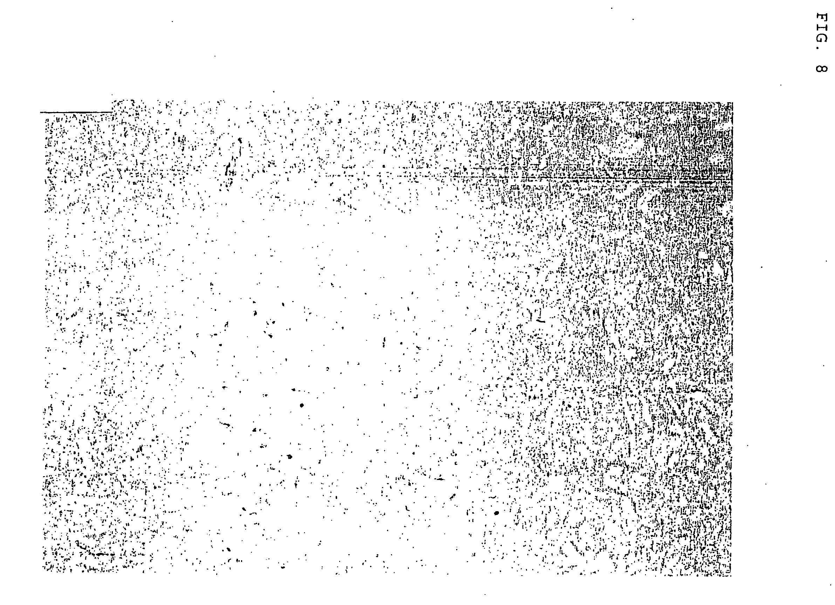

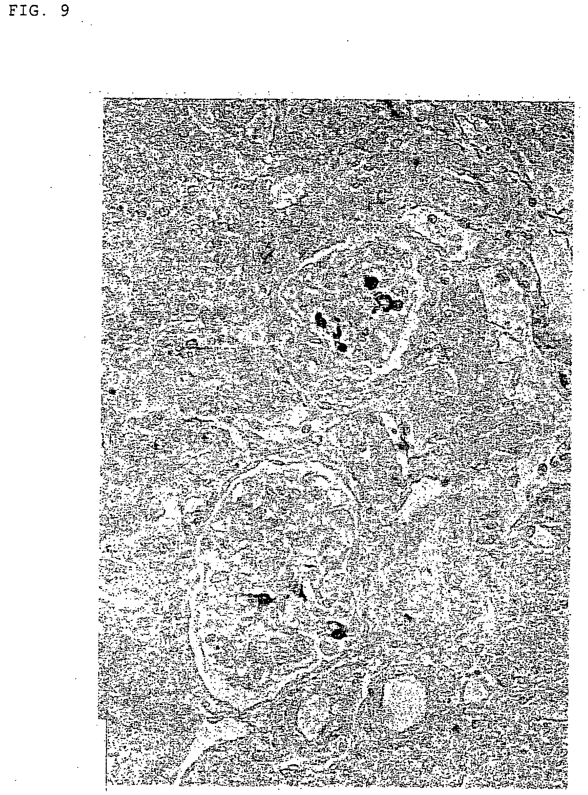

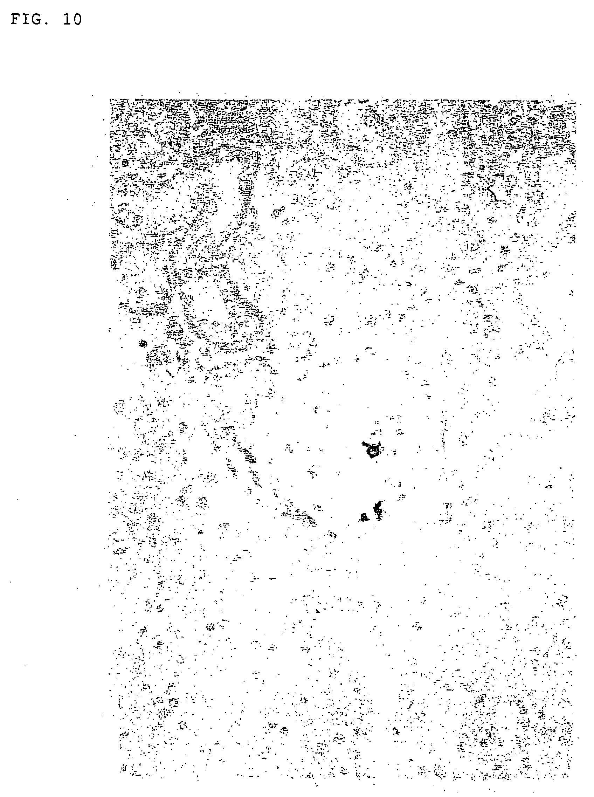

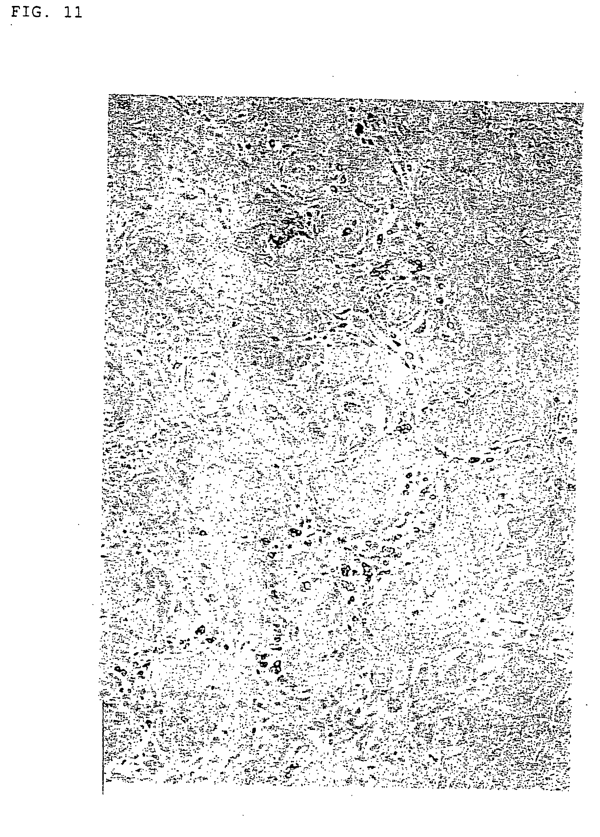

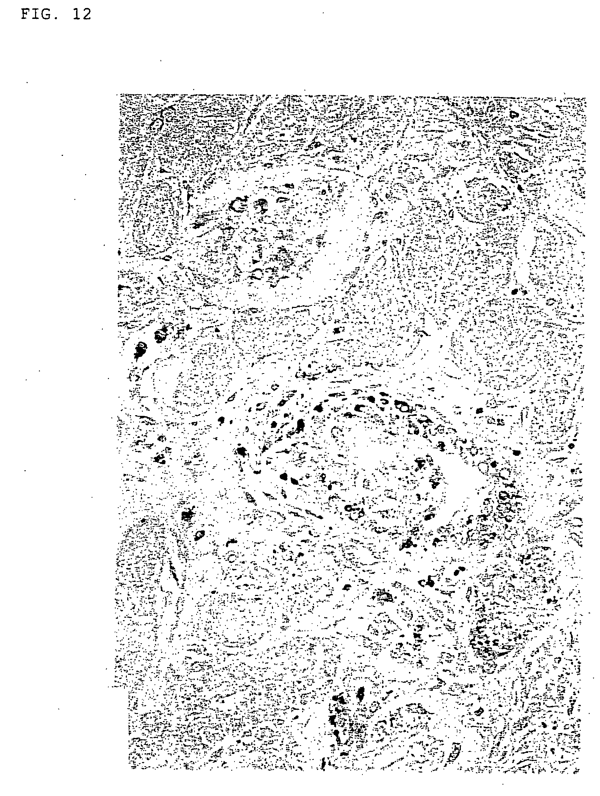

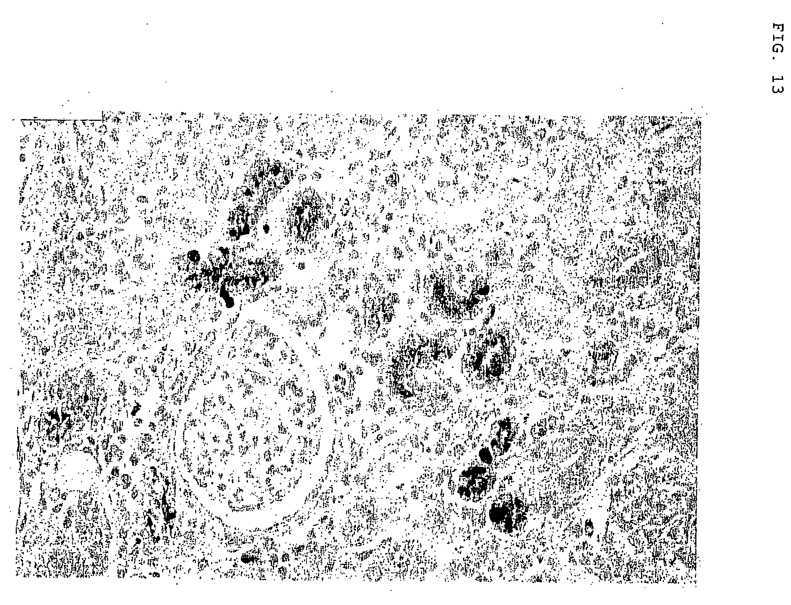

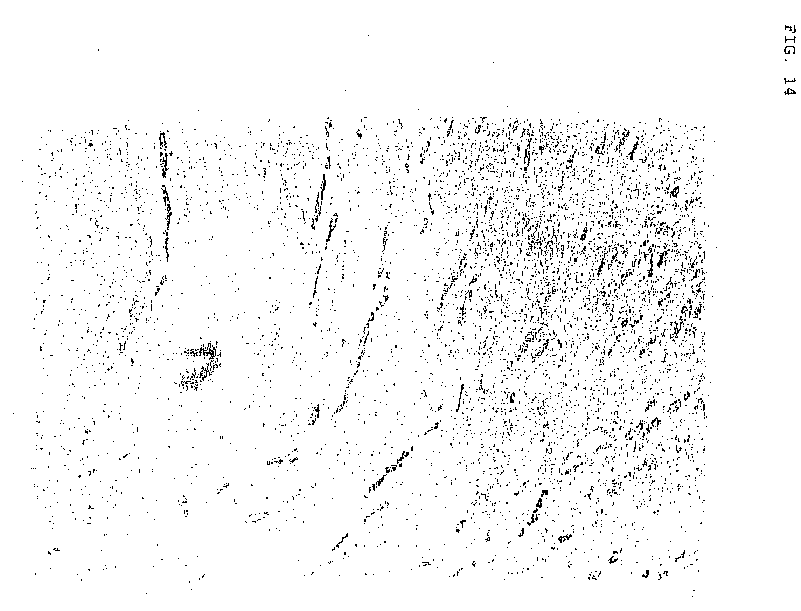

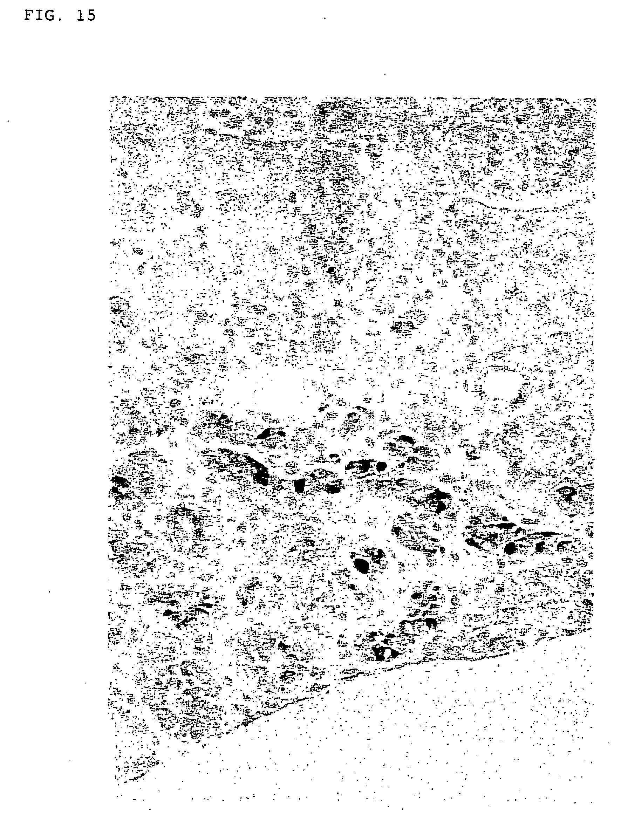

Six-week-old male SD rats were used, and administration was carried out from the renal artery throughout this experiment. The volume of virus liquid administered was 700 µl/individual; the titer of GFP/SeV was 2x 10⁹ pfu/ml in the liquid. As a result of immunohistochemical staining, GFP antibody-positive stains were detected for the samples prepared any sampling days after administration (7 days, Figs. 7 to 9; 14 days, Figs. 10 to 13). Almost no infiltration of macrophage was found near the interstitial cells and glomerular cells which were confirmed to be infected. In this administration experiment, GFP antibody-positive stains were recognized in renal tubular epithelial cells obtained in any sampling days (28 days, Figs. 14 and 15), although such stains were not detected 4 days after administration from the renal artery. In samples 28 days after administration, cell infiltration was recognized near GFP antibody-positive renal tubular cells in some cases. This shows a sign of the damage in renal tubular interstitium.

### Industrial Applicability

Although sufficiently highly efficient gene transfer into renal cells was not achieved with conventional vectors, the present invention using a Paramyxoviral vector made it possible to introduce genes into renal cells with very high efficiency by only short-time contact with the vector. The present invention provides a basic technology to achieve gene therapy targeting renal cells.

## Claims

1. A method of introducing a gene into a renal cell, wherein the method comprises contacting a Paramyxovirus vector with the renal cell.

2. The method according to claim 1, wherein the method further comprises administering the Paramyxovirus vector into a blood vessel.

3. The method according to claim 2, wherein the blood vessel is renal artery.

4. The method according to claim 1, wherein the method further comprises administering the Paramyxovirus vector into urinary tract.

5. The method according to any one of claims 1 to 4, wherein the renal cell is selected from the group consisting of an interstitial cell, a glomerular cell and a renal tubular cell.

6. The method according to any one of claims 1 to 5, wherein the Paramyxovirus is Sendai virus.

7. A Paramyxovirus vector for gene transfer into a renal cell.

8. A composition for gene transfer into a renal cell, wherein the composition comprises a Paramyxovirus vector or a cell comprising the vector.

9. The composition according to claim 8, wherein the composition is administered into a blood vessel.

10. The composition according to claim 9, wherein the blood vessel is renal artery.

11. The composition according to claim 8, wherein the composition is administered into urinary tract.

12. The composition according to any one of claims 8 to 11, wherein the renal cell is selected from the group consisting of an interstitial cell, a glomerular cell and a renal tubular cell.

13. The composition according to any one of claims 8 to 12, wherein the Paramyxovirus is Sendai virus.


SEQUENCE LISTING

<110> DNAVEC Research Inc.

<120> VIRUS VECTOR FOR INTRODUCING GENE INTO RENAL CELLS

<130> D3-A0005P

<140>
<141>

<150> JP 2000-197870
<151> 2000-06-27

<160> 10

<170> PatentIn Ver. 2.0

<210> 1
<211> 10
<212> DNA
<213> Artificial Sequence

<220>
<223> Description of Artificial Sequence: Artificially
      Synthesized Sequence

<400> 1
ctttcaccct                                                              10


<210> 2
<211> 15
<212> DNA
<213> Artificial Sequence


<220>
<223> Description of Artificial Sequence: Artificially
      Synthesized Sequence


<400> 2
tttttcttac tacgg                                                        15


<210> 3
<211> 18
<212> DNA
<213> Artificial Sequence


<220>
<223> Description of Artificial Sequence: Artificially
      Synthesized Sequence


<400> 3

cggccgcaga tcttcacg 18

<210> 4

<211> 18

<212> DNA

<213> Artificial Sequence

<220>

<223> Description of Artificial Sequence: Artificially

Synthesized Sequence

<400> 4

atgcatgccg gcagatga 18

<210> 5

<211> 18

<212> DNA

<213> Artificial Sequence

<220>

<223> Description of Artificial Sequence: Artificially

Synthesized Primer Sequence

<400> 5

gttgagtact gcaagagc 18

<210> 6

<211> 42

<212> DNA

<213> Artificial Sequence

<220>

<223> Description of Artificial Sequence: Artificially
      Synthesized Primer Sequence

<400> 6

tttgccggca tgcatgtttc ccaaggggag agttttgcaa cc                42

<210> 7

<211> 18

<212> DNA

<213> Artificial Sequence

<220>

<223> Description of Artificial Sequence: Artificially
      Synthesized Primer Sequence

<400> 7

atgcatgccg gcagatga                                           18

<210> 8

<211> 21

<212> DNA

<213> Artificial Sequence

<220>

<223> Description of Artificial Sequence: Artificially

Synthesized Primer Sequence

<400> 8

tgggtgaatg agagaatcag c 21

<210> 9

<211> 30

<212> DNA

<213> Artificial Sequence

<220>

<223> Description of Artificial Sequence: Artificially

Synthesized Primer Sequence

<400> 9

atgcatatgg tgatgcggtt ttggcagtac 30

<210> 10

<211> 30

<212> DNA

<213> Artificial Sequence

<220>

<223> Description of Artificial Sequence: Artificially
      Synthesized Primer Sequence

<400> 10
tgccggctat tattacttgt acagctcgtc                                        30